# EUROPEAN PATENT APPLICATION

(11) **EP 4 606 275 A1**
(43) Date of publication of application: **27.08.2025**
(21) Application number: 23879610.6
(22) Date of filing: 03.10.2023
(51) Int. Cl.: A47J 27/00, G06Q 50/10, H04M 11/00

(54) **CONTROL METHOD, INFORMATION PROVISION METHOD, CONTROL SYSTEM, INFORMATION PROVISION SYSTEM, AND PROGRAM**

(30) Priority: 21.10.2022 JP 2022169311
(71) Applicant: Panasonic Intellectual Property Management Co., Ltd., Kadoma-shi, Osaka 571-0057 (JP)
(72) Inventor: YAMAJI Satoru, Kadoma-shi, Osaka 571-0057 (JP); TSUJI Kiyotaka, Kadoma-shi, Osaka 571-0057 (JP); KONDO Kenji, Kadoma-shi, Osaka 571-0057 (JP); OKUMURA Yasuaki, Kadoma-shi, Osaka 571-0057 (JP)
(74) Representative: Grünecker Patent- und Rechtsanwälte PartG mbB
(86) International application number: PCT/JP2023/036100
(87) International publication number: WO 2024/084963

(57) **Abstract**

A control method includes acquiring (step S110) target nutritional information; acquiring (step S120) initial cooking parameter information used for cooking by a cooking device, based on the acquired target nutritional information; acquiring (step S150) cooking-in-progress nutritional information of an item to be cooked when the cooking by the cooking device is being performed; changing (step S180) the initial cooking parameter information to modified cooking parameter information including one or more modified cooking parameters, based on the target nutritional information and the cooking-in-progress nutritional information; and outputting (step S190) control information including the modified cooking parameter information.

## Description

### Technical Field

The present disclosure relates to a control method related to cooking, an information providing method related to cooking, or the like.

### Background Art

In the related art, a cooking method for improving convenience of cooking has been proposed as a control method related to cooking (see, for example, PTL 1). In this cooking method, increase or decrease information indicating an increase or a decrease in food ingredients being cooked is acquired from a cooking appliance, the increase or decrease information is converted into calories, and the heating time of the cooking appliance is adjusted according to the calories. For example, the increase or decrease information is obtained from an image captured by a camera. It is therefore possible to save the time for a user to adjust the heating time in accordance with an increase or a decrease in food ingredients being cooked.

In addition, a cooking assistance system for assisting in cooking has been proposed (see, for example, PTL 2). The cooking assistance system is configured as smart glasses. The smart glasses identify a size of food ingredient pieces generated by a cook cutting food ingredients, estimate an optimal heating and cooking time for the food ingredient pieces based on the size of the food ingredient pieces, and display the estimated optimal heating and cooking time. That is, the control method related to cooking using the smart glasses involves identifying the size of food ingredient pieces, estimating the heating and cooking time, and so on. Accordingly, even if the cook cuts the food ingredients into pieces whose sizes are different from those specified in a recipe, the cook can easily cook the food ingredients using a heating and cooking time suitable for the size of the food ingredient pieces obtained from the cutting of the food ingredients.

### Citation List

### Patent Literature

PTL 1: Japanese Unexamined Patent Application Publication No. 2020-159581
PTL 2: Japanese Patent No. 6692960

### Summary of Invention

However, the control methods in PTLs described above have the issue of not providing appropriate control over the nutrition of a dish obtained by cooking.

The present disclosure provides a control method and the like that can appropriately control the nutrition of a dish.

A control method according to one aspect of the present disclosure is a control method executed by a computer to control a cooking device that cooks an item to be cooked, the control method including acquiring target nutritional information indicating a target for one or more numerical values related to nutrition of a dish obtained by cooking the item to be cooked; acquiring cooking parameter information including one or more cooking parameters used for cooking by the cooking device, based on the acquired target nutritional information; acquiring cooking-in-progress nutritional information indicating the one or more numerical values related to the nutrition of the item to be cooked when the cooking by the cooking device is being performed; changing the cooking parameter information to modified cooking parameter information including one or more modified cooking parameters, based on the target nutritional information and the cooking-in-progress nutritional information; and outputting a control signal including the modified cooking parameter information.

This comprehensive or specific aspect may be implemented by an apparatus, a system, an integrated circuit, or a computer-readable recording medium, or may be implemented by any combination of an apparatus, a system, a method, an integrated circuit, a computer program, and a computer-readable recording medium. The computer-readable recording medium includes, for example, a non-volatile recording medium such as a compact disc-read only memory (CD-ROM). The computer-readable recording medium may be a non-transitory recording medium.

According to the present disclosure, the nutrition of a dish can be appropriately controlled.

Further advantages and effects of one aspect of the present disclosure will become apparent from the description and the drawings. Such advantages and/or effects may be provided by some embodiments and configurations described in the description and the drawings, but not all of the configurations are required.

### Brief Description of Drawings

[Fig. 1] Fig. 1 is a diagram illustrating an example of a configuration of an information processing system according to an embodiment.
[Fig. 2] Fig. 2 is a block diagram illustrating an example of a functional configuration of an operation terminal and a cooking device according to the embodiment.
[Fig. 3] Fig. 3 is a block diagram illustrating an example of a functional configuration of a server according to the embodiment.
[Fig. 4] Fig. 4 is a diagram illustrating details of types of nutritional information stored in a third storage unit according to the embodiment.
[Fig. 5] Fig. 5 is a block diagram illustrating an example of a detailed functional configuration of a server control unit according to the embodiment.
[Fig. 6] Fig. 6 is a diagram illustrating an example of dish list information, recipe information, basic cooking parameter information, and basic nutritional information according to the embodiment.
[Fig. 7] Fig. 7 is a diagram illustrating an example of image data, chemical analysis data, and weight data included in cooking state information according to the embodiment.
[Fig. 8] Fig. 8 is a diagram illustrating an example of screens displayed on the operation terminal according to the embodiment.
[Fig. 9] Fig. 9 is a diagram illustrating another example of screens displayed on the operation terminal according to the embodiment.
[Fig. 10] Fig. 10 is a sequence diagram illustrating an example of a processing operation of the information processing system according to the embodiment.
[Fig. 11] Fig. 11 is a flowchart illustrating an example of a processing operation of the server according to the embodiment.
[Fig. 12] Fig. 12 illustrates flowcharts illustrating portions of the processing operation of the server according to the embodiment in detail.
[Fig. 13] Fig. 13 is a flowchart illustrating an example of constructing a first learning model according to the embodiment.
[Fig. 14] Fig. 14 is a flowchart illustrating an example of constructing a second learning model according to the embodiment.
[Fig. 15] Fig. 15 is a flowchart illustrating an example of constructing a third learning model according to the embodiment.
[Fig. 16] Fig. 16 is a flowchart illustrating an example of constructing a fourth learning model according to the embodiment.
[Fig. 17] Fig. 17 illustrates an example of the first learning model.
[Fig. 18] Fig. 18 illustrates an example of the second learning model.
[Fig. 19] Fig. 19 illustrates an example of the third learning model.
[Fig. 20] Fig. 20 illustrates an example of the fourth learning model.

### Description of Embodiments

### (Underlying Knowledge Forming Basis of the Present Disclosure)

When cooking, a cook may take into consideration the nutritional components of a dish obtained by cooking. Examples of the dish include bouillon and braised pork belly. The nutritional components include, for example, energy and amounts of nutrients. The nutrients include, for example, protein, fat, and carbohydrate.

When cooking, the cook prepares an item to be cooked made of one or more food ingredients according to a recipe for the dish and cooks the item to be cooked, thereby being able to create a dish with energy according to the recipe or a dish containing nutrients in the amounts specified in the recipe.

However, the cook may desire energy and amounts of nutrients different from those specified in the recipe. Furthermore, even if the cook desires the energy and amounts of nutrients specified in the recipe, it may be difficult to prepare the food ingredients in the exact amounts specified in the recipe, or it may be difficult to prepare the respective food ingredients in the proportions specified in the recipe. Even if the respective food ingredients are prepared in the proportions specified in the recipe, the energy and the amounts of nutrients may fluctuate during the cooking process. **In** such cases, the temperature, the pressure, the time, and the like of a cooking device for cooking the item to be cooked are difficult to appropriately set in the cooking device in accordance with the desire of the cook.

Accordingly, a control method according to a first aspect of the present disclosure is a control method executed by a computer to control a cooking device that cooks an item to be cooked, the control method including acquiring target nutritional information indicating a target for one or more numerical values related to nutrition of a dish obtained by cooking the item to be cooked; acquiring cooking parameter information including one or more cooking parameters used for cooking by the cooking device, based on the acquired target nutritional information; acquiring cooking-in-progress nutritional information indicating the one or more numerical values related to the nutrition of the item to be cooked when the cooking by the cooking device is being performed; changing the cooking parameter information to modified cooking parameter information including one or more modified cooking parameters, based on the target nutritional information and the cooking-in-progress nutritional information; and outputting a control signal including the modified cooking parameter information. The one or more nutrients may include protein, fat, and the like, and may include, for example, health components such as melanoidin and gelatin.

With this configuration, for example, target nutritional information desired by a cook is acquired, and cooking parameter information is acquired as, for example, initial cooking parameter information, based on the target nutritional information. Then, for example, in response to transmission of the cooking parameter information to the cooking device, cooking according to the cooking parameter information is started by the cooking device. Thereafter, the cooking parameter information used by the cooking device is changed using one or more numerical values (i.e., energy and the like) related to the nutrition of the item to be cooked when cooking is being performed. Accordingly, cooking according to the modified cooking parameter information makes it possible to bring the energy and the like of a finally prepared dish closer to the energy and the like indicated by the target nutritional information. **In** other words, it is possible to bring the energy and the like of a finally prepared dish closer to the energy and the like desired by the cook. That is, even if the energy and the like desired by the cook are different from the energy and the like specified in the basic recipe, or even if the amount of the item to be cooked is not as specified in the recipe, the energy and the like of a final dish can be brought closer to the energy and the like desired by the cook. As a result, it is possible to appropriately control the nutrition of the dish without requiring the cook to adjust cooking parameters.

**In** a control method according to a second aspect, dependent on the first aspect, the acquiring of the cooking-in-progress nutritional information may acquire, as first nutritional information, the cooking-in-progress nutritional information at a time point when a first time period elapses after a start of the cooking by the cooking device, and acquire, as second nutritional information, the cooking-in-progress nutritional information at a time point when a second time period elapses after the first time period has elapsed since the start of the cooking by the cooking device, and the changing of the cooking parameter information may change the cooking parameter information to the modified cooking parameter information, based on the target nutritional information and a difference between the first nutritional information and the second nutritional information.

With this configuration, the cooking parameter information is changed to the modified cooking parameter information based on the amount of change in the cooking-in-progress nutritional information and the target nutritional information. Thus, by taking into consideration the tendency of changes in energy and the like caused by the cooking according to the cooking parameter information, the energy and the like of a final dish can be effectively brought closer to the energy and the like desired by the cook. The first time period may be 0 hours. In this case, the first nutritional information indicates one or more numerical values related to the nutrition of the item to be cooked at the time point of the start of cooking.

A control method according to a third aspect, dependent on the first or second aspect, may further include acquiring third nutritional information indicating the one or more numerical values related to the nutrition of the dish obtained by cooking according to the modified cooking parameter information by the cooking device.

With this configuration, information indicating the energy and the like of a finally prepared dish is estimated as the third nutritional information. Thus, for example, by presenting the third nutritional information to the cook, the cook can check whether a dish containing the energy and the like desired by the cook will be prepared, or how close the energy of a finally prepared dish is to the desired one.

In a control method according to a fourth aspect, dependent on any one of the first to third aspects, the changing of the cooking parameter information may acquire candidates for the modified cooking parameter information, based on the target nutritional information and the cooking-in-progress nutritional information, and select, from among the candidates, a candidate whose difference from the cooking parameter information is less than or equal to a threshold or a candidate closest to the cooking parameter information, as the modified cooking parameter information.

With this configuration, even if, for example, pieces of new cooking parameter information are output as candidates in response to an input of the target nutritional information and the cooking-in-progress nutrition information to a learning model, a candidate that is not greatly different from the original cooking parameter information (i.e., initial cooking parameter information) is adopted as modified cooking parameter information. Thus, the processing load on the cooking device caused by the change of the cooking parameter information can be reduced.

In a control method according to a fifth aspect, dependent on the third aspect, the acquiring of the third nutritional information may acquire the third nutritional information at least by inputting the modified cooking parameter information to a learning model, and the learning model may be trained by machine learning such that, at least in response to an input of one or more cooking parameters changed during the cooking by the cooking device, the one or more numerical values related to the nutrition of the dish obtained by cooking according to the one or more cooking parameters by the cooking device are output.

With this configuration, the learning model is used for the acquisition of the third nutritional information. Thus, the third nutritional information with a high degree of accuracy can be acquired.

In a control method according to a sixth aspect, dependent on any one of the first to fifth aspects, the acquiring of the cooking-in-progress nutritional information may acquire the cooking-in-progress nutritional information by inputting, to a learning model, at least one of an image of the item to be cooked, a weight of the item to be cooked, and an amount of a chemical component contained in the item to be cooked when the cooking by the cooking device is being performed.

With this configuration, the learning model is used for the acquisition of the cooking-in-progress nutritional information. Thus, cooking-in-progress nutritional information with a high degree of accuracy can be acquired.

In a control method according to a seventh aspect, dependent on the sixth aspect, the learning model may be trained by machine learning such that, in response to an input of at least one of an image of one or more food ingredients being cooked by the cooking device, a weight of the one or more food ingredients, and an amount of a chemical component contained in the one or more food ingredients, the one or more numerical values related to the nutrition of the one or more food ingredients are output.

With this configuration, when at least one of an image of the item to be cooked, a weight of the item to be cooked, and an amount of a chemical component contained in the item to be cooked is input to the learning model trained by machine learning, cooking-in-progress nutritional information with a high degree of accuracy can be acquired from the learning model.

In a control method according to an eighth aspect, dependent on any one of the first to seventh aspects, the one or more cooking parameters may include a parameter indicating a temperature used for the cooking by the cooking device, and a parameter indicating a time used for the cooking by the cooking device.

With this configuration, the nutrition of a dish obtained by, for example, grilling can appropriately controlled.

In a control method according to a ninth aspect, dependent on the eighth aspect, the one or more cooking parameters may further include a parameter indicating a pressure used for the cooking by the cooking device.

With this configuration, the nutrition of a dish obtained by, for example, steaming or boiling under pressure can appropriately controlled.

Further, an information providing method according to one aspect of the present disclosure is an information providing method for a computer to provide information related to cooking of an item to be cooked by a cooking device, the information providing method including receiving, in response to an operation by a user, target nutritional information indicating a target for one or more numerical values related to nutrition of a dish obtained by cooking the item to be cooked; and outputting final nutritional information indicating the one or more numerical values related to the nutrition of the dish, the final nutritional information being derived based on the target nutritional information and cooking-in-progress nutritional information, the cooking-in-progress nutritional information indicating the one or more numerical values related to the nutrition of the item to be cooked when the item to be cooked is being cooked by the cooking device.

With this configuration, for example, when dish information indicating a dish desired by a cook who is a user of the computer and the target nutritional information desired by the cook are received, one or more numerical values (i.e., energy and the like) related to the nutrition of a finally prepared dish in accordance with the received information can be presented to the cook. This allows the cook to appropriately grasp the nutrition of the dish obtained by cooking. That is, the cook can check whether a dish containing the energy and the like desired by the cook will be prepared, or how close the energy of a finally prepared dish is to the desired one.

The processing operations included in the control method and the information providing method described above are executed by the computer.

Hereinafter, embodiments of the present disclosure will be described with reference to the drawings. Each of the embodiments described below represents a specific example of the present disclosure. Accordingly, numerical values, shapes, materials, components, the arrangement and connection of the components, and the like described in the following embodiments are examples and are not intended to limit the present disclosure. Thus, among the components in the following embodiments, components not recited in any one of the independent claims, which indicate the broadest concepts, are described as optional components.

Note that the drawings are schematic and not necessarily drawn to scale. In addition, in the drawings, substantially the same components are denoted by the same reference numerals, and redundant descriptions thereof will be omitted or simplified. The operations and effects of the control method and the information providing method described above are also implemented in a system and a program in a similar manner.

### (Embodiment 1)

### [Overall Configuration of Information Processing System]

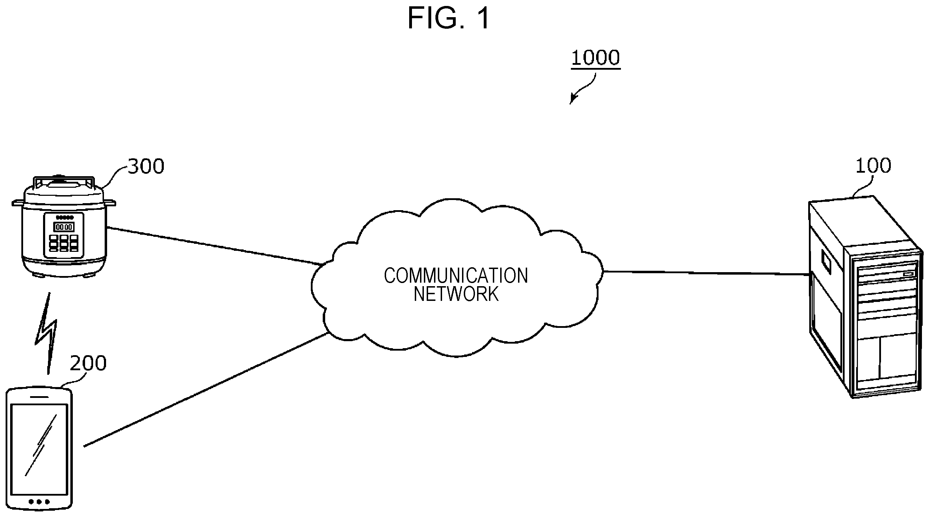

Fig. 1 is a diagram illustrating an example of a configuration of an information processing system 1000 according to the present embodiment.

The information processing system 1000 includes a server 100, an operation terminal 200, and a cooking device 300, which are connected to each other via a communication network such as the Internet.

The cooking device 300 is a device for cooking an item to be cooked made of one or more food ingredients and the like to create a dish, and adjusts pressure, temperature, and time as cooking parameters to perform cooking. In the present embodiment, as an example, the cooking device 300 is configured as a pressure cooker.

The number of cooking parameters adjustable by the cooking device 300 is not limited to three, and may be one or two, or may be four or more. The cooking device 300 may be a device that boils or steams an item to be cooked, or may be an induction heating (IH) cooking device or a temperature-adjustable container. The temperature-adjusted container controls temperature and time to perform fermentation and maturation of soy sauce, miso, and the like as a cooking process.

The cooking device 300 may also be a cooking device that uses a liquid or gas in a region just before the critical point (the point where the temperature is 374°C and the pressure is 22 MPa). Such a liquid (e.g., water) or gas has, for example, a pressure exceeding 0.2 MPa and a temperature exceeding 120°C. Such types of cooking devices include a pressure cooker type, a superheated steam type, and a frying pan type. A cooking device of the pressure cooker type is capable of independently controlling temperature and pressure in a closed system, and uses the liquid to perform cooking such as boiling and stewing. A cooking device of the superheated steam type sprays the gas (e.g., steam) onto the item to be cooked from a unit capable of controlling at least the temperature of the temperature and the pressure in a closed system or an open system, and performs cooking such as steaming and grilling. A cooking device of the frying pan type includes an IH cooking device and a frying pan placed on the IH cooking device, and performs grilling by controlling temperature and time. Such cooking with a liquid or a gas can promote a hydrolysis reaction.

The operation terminal 200 is configured as, for example, a smartphone or a tablet terminal. The operation terminal 200 is operated by a cook to cook using the cooking device 300. The cook may be a user who uses the cooking device 300.

The server 100 controls the cooking device 300 in accordance with an input operation performed on the operation terminal 200 by the cook. That is, the server 100 is a computer that executes a control method for controlling the cooking device 300 that cooks an item to be cooked. In other words, the server 100 is a control system that controls the cooking device 300 that cooks an item to be cooked.

### [Configuration of Server, Operation Terminal, and Cooking Device]

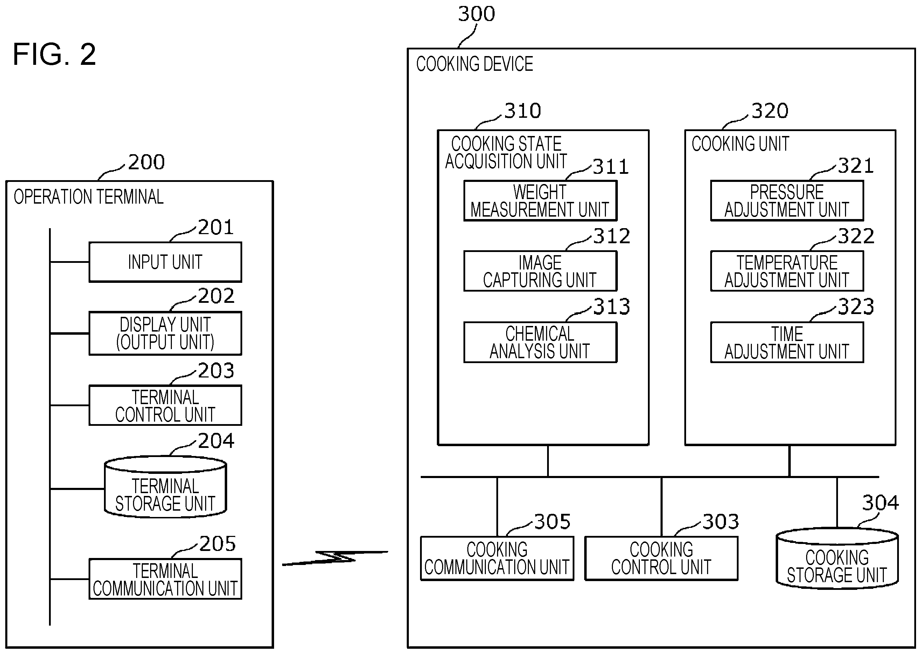

Fig. 2 is a block diagram illustrating an example of a functional configuration of the operation terminal 200 and the cooking device 300.

The operation terminal 200 is a computer that executes an information providing method for providing information related to cooking of an item to be cooked by the cooking device 300, and includes an input unit 201, a display unit 202, a terminal control unit 203, a terminal storage unit 204, and a terminal communication unit 205. The operation terminal 200 could also be considered an information providing system that provides information related to cooking of an item to be cooked by the cooking device 300.

The display unit 202 is a device that displays an image, and is, for example, a liquid crystal display, an organic EL (Electro-Luminescence) display, or the like. Note that the display unit 202 is not limited to these, and may be any device that can display an image. Moreover, the display unit 202 in the present embodiment could also be considered an output unit that outputs information related to cooking. In the present embodiment, the display unit 202 is provided in the operation terminal 200 as an example of an output unit that outputs information related to cooking. However, an audio output unit (e.g., a speaker or the like) that outputs the information by voice may also be provided as the output unit. Both the display unit 202 and the audio output unit may be provided as an output unit.

The input unit 201 is configured as, for example, a touch sensor disposed on the display unit 202 such that when the cook touches an image such as an icon displayed on the display unit 202, the touch sensor receives an input operation corresponding to the image. The input unit 201 may also include a button, and when the cook presses the button, the input unit 201 may receive an input operation corresponding to the button.

The terminal communication unit 205 communicates with the cooking device 300 wirelessly or via wired connection. The wireless communication may be performed using Wi-Fi (registered trademark), Bluetooth (registered trademark), ZigBee (registered trademark), or a specific low-power radio. The terminal communication unit 205 further communicates with the server 100 via the communication network described above. The terminal communication unit 205 may communicate directly with the server 100, or may communicate with the server 100 via the cooking device 300.

The terminal storage unit 204 is a recording medium for storing various types of information, data, programs, and the like. The terminal storage unit 204 is a hard disk drive, a random access memory (RAM), a read only memory (ROM), a semiconductor memory, or the like. The terminal storage unit 204 may be either volatile or non-volatile.

The terminal control unit 203 controls the input unit 201, the display unit 202, the terminal communication unit 205, and the like by, for example, reading and executing a program stored in the terminal storage unit 204.

The cooking device 300 includes a cooking control unit 303, a cooking storage unit 304, a cooking communication unit 305, a cooking state acquisition unit 310, and a cooking unit 320.

The cooking unit 320 cooks an item to be cooked made of one or more food ingredients and the like by applying physical action to the item to be cooked in accordance with cooking parameter information including three cooking parameters such as temperature, pressure, and time. The cooking unit 320 includes a pressure adjustment unit 321, a temperature adjustment unit 322, and a time adjustment unit 323.

The pressure adjustment unit 321 adjusts the pressure to be applied to the item to be cooked in accordance with the cooking parameter indicating pressure. The temperature adjustment unit 322 adjusts the temperature to be applied to the item to be cooked in accordance with the cooking parameter indicating temperature. The time adjustment unit 323 adjusts the duration of the pressure or the temperature adjusted by the pressure adjustment unit 321 and the temperature adjustment unit 322 in accordance with the cooking parameter indicating time.

The cooking state acquisition unit 310 acquires cooking state information indicating the state of the item to be cooked. The cooking state acquisition unit 310 includes a weight measurement unit 311, an image capturing unit 312, and a chemical analysis unit 313. The weight measurement unit 311 measures the weight of the item to be cooked placed in the cooking device 300. Then, the weight measurement unit 311 outputs weight data indicating the measured weight. The image capturing unit 312 is, for example, a camera, and captures an image of the item to be cooked placed in the cooking device 300. Then, the image capturing unit 312 outputs image data obtained by capturing the image of the item to be cooked. The image capturing unit 312 is, for example, an image sensor that is water-resistant and has high performance in dark places. The chemical analysis unit 313 analyzes the chemical components of the item to be cooked placed in the cooking device 300 by, for example, a liquid chromatographic method, a gas chromatographic method, or the like. Then, the chemical analysis unit 313 outputs chemical analysis data obtained by analyzing the chemical components. The chromatographic methods described above are not limiting, and the chemical analysis unit 313 may analyze the chemical components of the item to be cooked by using any other method such as an ether extraction method. The chemical analysis unit 313 may analyze the chemical components of the item to be cooked placed in the cooking device 300, based on, for example, information detected by an odor sensor. The odor sensor includes odor detection elements. Specifically, the odor sensor includes a first odor detection element to an n-th odor detection element (n is an integer greater than or equal to 2). Output signals from the first odor detection element to the n-th odor detection element may be subjected to machine learning and analysis to determine one or more chemical components contained in the item to be cooked, and the amount of each of the one or more chemical components.

The cooking state information includes the weight data, the image data, and the chemical analysis data described above.

The cooking communication unit 305 communicates with the operation terminal 200 wirelessly or via wired connection. As described above, the wireless communication may be performed using Wi-Fi, Bluetooth, or the like. The cooking communication unit 305 further communicates with the server 100 via the communication network described above. The cooking communication unit 305 may communicate directly with the server 100, or may communicate with the server 100 via the operation terminal 200.

The cooking storage unit 304 is a recording medium for storing various types of information, data, programs, and the like. Like the terminal storage unit 204, the cooking storage unit 304 is a hard disk drive, a RAM, a ROM, or the like. The cooking storage unit 304 may be either volatile or non-volatile.

The cooking control unit 303 controls the cooking communication unit 305, the cooking state acquisition unit 310, the cooking unit 320, and the like by, for example, reading and executing a program stored in the cooking storage unit 304.

In the present embodiment, the cooking device 300 performs cooking in accordance with three cooking parameters. However, the cooking device 300 may perform cooking in accordance with one or two cooking parameters, or may perform cooking in accordance with four or more cooking parameters.

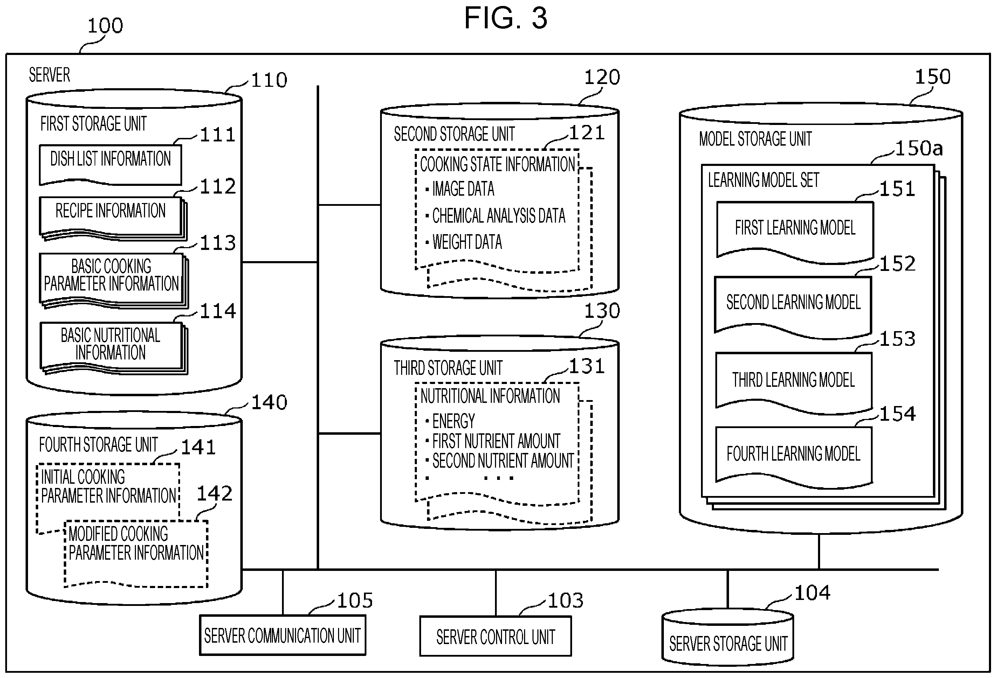

Fig. 3 is a block diagram illustrating an example of a functional configuration of the server 100.

The server 100 is a computer that controls the cooking device 300 that cooks an item to be cooked, and includes a server control unit 103, a server storage unit 104, a server communication unit 105, a first storage unit 110, a second storage unit 120, a third storage unit 130, a fourth storage unit 140, and a model storage unit 150. The server storage unit 104, the first storage unit 110, the second storage unit 120, the third storage unit 130, the fourth storage unit 140, and the model storage unit 150 are each a recording medium and each a hard disk drive, a RAM, a ROM, or the like, like the terminal storage unit 204 and the cooking storage unit 304. These recording media may be either volatile or non-volatile. The server 100 is also referred to as a control system.

The first storage unit 110 stores dish list information 111, recipe information 112, basic cooking parameter information 113, and basic nutritional information 114. The dish list information 111 indicates the respective names of dishes (hereinafter referred to as dish names). The recipe information 112 indicates, for each dish name indicated by the dish list information 111, one or more ingredients (also referred to as food ingredients) used in the dish with the dish name, and the amount of each of the one or more ingredients. The basic cooking parameter information 113 indicates, for each dish name indicated by the dish list information 111, one or more basic cooking parameters used by the cooking unit 320 of the cooking device 300 to prepare the dish with the dish name. The basic nutritional information 114 indicates, for each dish name indicated by the dish list information 111, one or more numerical values related to the nutrition included in the dish prepared according to the recipe information 112 and the basic cooking parameter information 113 corresponding to the dish name.

The second storage unit 120 is a recording medium for storing cooking state information 121 obtained by the cooking state acquisition unit 310 of the cooking device 300. The cooking state information 121 includes image data, chemical analysis data, and weight data that are obtained at substantially the same timing.

The third storage unit 130 is a recording medium for storing nutritional information 131 indicating one or more numerical values related to the nutrition included in a dish or an item to be cooked. Specifically, the nutritional information 131 indicates, as the one or more numerical values related to the nutrition, energy and the amount of each of one or more nutrients. The amount of a nutrient is also referred to as a nutrient amount. For example, the nutritional information 131 indicates a first nutrient amount, a second nutrient amount, etc. The first nutrient amount may be an amount of protein, and the second nutrient amount may be an amount of fat. The nutrient amount may also be the amount of a healthy ingredient such as melanoidin or gelatin. While, in the present embodiment, the nutritional information 131 indicates energy and the amount of each of one or more nutrients, the nutritional information 131 may indicate either of them. That is, the nutritional information 131 may indicate at least one of energy and the amount of each of one or more nutrients, or may indicate only one nutrient amount. In the present disclosure, "energy" may mean "energy value". The unit of "energy value" may be a calorie.

The fourth storage unit 140 is a recording medium for storing initial cooking parameter information 141 and modified cooking parameter information 142. The initial cooking parameter information 141 and the modified cooking parameter information 142 are each cooking parameter information indicating one or more cooking parameters used for cooking by the cooking device 300. The initial cooking parameter information 141 indicates one or more cooking parameters used when the cooking by the cooking device 300 is started. The modified cooking parameter information 142 is information modified or changed from the initial cooking parameter information 141. That is, the modified cooking parameter information 142 includes one or more modified cooking parameters obtained by modifying or changing one or more cooking parameters included in the initial cooking parameter information 141.

The model storage unit 150 stores, for each dish name indicated by the dish list information 111, a learning model set 150a corresponding to the dish name. The learning model set 150a includes a first learning model 151, a second learning model 152, a third learning model 153, and a fourth learning model 154. These learning models are models such as neural networks trained via machine learning, for example.

The first learning model 151 is a model for deriving the initial cooking parameter information 141 described above. The second learning model 152 is a model for deriving, as cooking-in-progress nutritional information, the nutritional information 131 of the item to be cooked when the cooking by the cooking device 300 is being performed. The third learning model 153 is a model for deriving the modified cooking parameter information 142 described above. The fourth learning model 154 is a model for deriving, as third nutritional information, final nutritional information 131 of a dish obtained by cooking according to the initial cooking parameter information 141 and the modified cooking parameter information 142 by the cooking device 300.

The server communication unit 105 communicates with the operation terminal 200 and the cooking device 300 via the communication network described above. The server storage unit 104 is a recording medium for storing various types of information, data, programs, and the like.

The server control unit 103 controls the server communication unit 105 and the like by, for example, reading and executing a program stored in the server storage unit 104. For example, the server control unit 103 acquires the cooking state information 121 from the cooking device 300 via the server communication unit 105, and stores the cooking state information 121 in the second storage unit 120. The server control unit 103 further acquires the nutritional information 131, the initial cooking parameter information 141, and the modified cooking parameter information 142, which are derived using the learning model set 150a. Then, the server control unit 103 stores the acquired nutritional information 131 in the third storage unit 130, and stores the acquired initial cooking parameter information 141 and modified cooking parameter information 142 in the fourth storage unit 140.

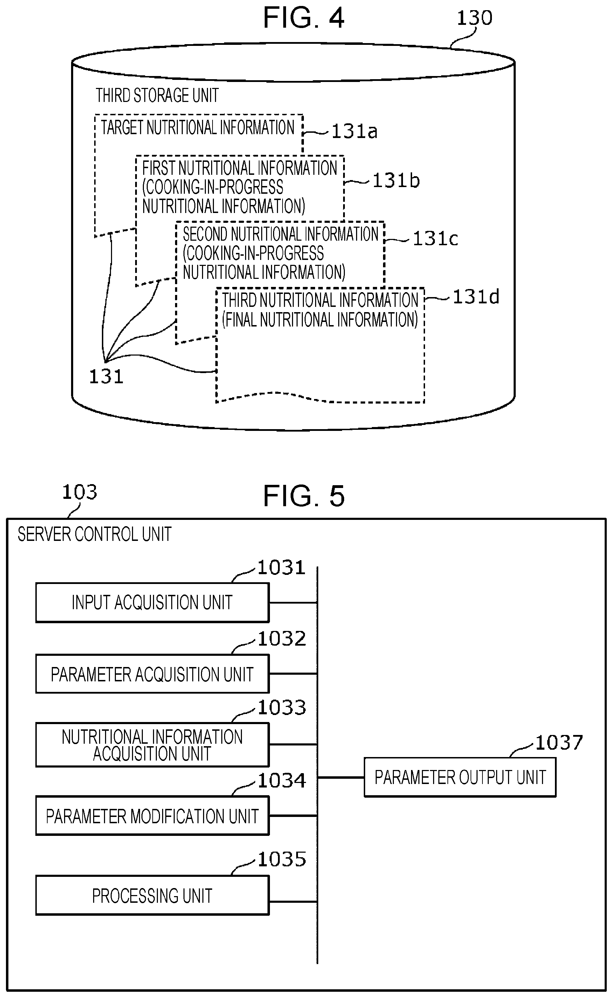

Fig. 4 is a diagram illustrating details of types of nutritional information 131 stored in the third storage unit 130.

As illustrated in Fig. 4, the third storage unit 130 stores types of nutritional information 131. The types of nutritional information 131 are target nutritional information 131a, first nutritional information 131b, second nutritional information 131c, and third nutritional information 131d, which will be described below. The target nutritional information 131a is nutritional information 131 that is a target for the cook. Each of the first nutritional information 131b and the second nutritional information 131c is the nutritional information 131 of the item to be cooked when cooking is being performed, and is also referred to as cooking-in-progress nutritional information. The third nutritional information 131d is the nutritional information 131 of a dish created by cooking, and is also referred to as final nutritional information.

Fig. 5 is a block diagram illustrating an example of a detailed functional configuration of the server control unit 103.

The server control unit 103 includes an input acquisition unit 1031, a parameter acquisition unit 1032, a nutritional information acquisition unit 1033, a parameter modification unit 1034, a processing unit 1035, and a parameter output unit 1037.

The input acquisition unit 1031 acquires input information from the operation terminal 200 via the server communication unit 105. The input information is information that is received by the terminal control unit 203 in response to an input operation performed on the input unit 201 of the operation terminal 200 by the cook. The cook performs an input operation on the input unit 201 of the operation terminal 200 to input, for example, the name of a dish to be prepared by the cooking device 300 (i.e., a dish name) and a numerical value related to target nutrition. As a result, the terminal control unit 203 receives input information including dish information indicating the dish name and the target nutritional information 131a indicating the numerical value related to the target nutrition. The input acquisition unit 1031 acquires the input information from the terminal control unit 203 via the terminal communication unit 205 and the server communication unit 105.

That is, the input acquisition unit 1031 in the present embodiment acquires dish information indicating a dish obtained by cooking an item to be cooked, and (b) the target nutritional information 131a indicating a target for one or more numerical values related to nutrition.

The parameter acquisition unit 1032 acquires cooking parameter information indicating one or more cooking parameters by using the input information and the learning model set 150a stored in the model storage unit 150. That is, the parameter acquisition unit 1032 uses the learning model set 150a to acquire cooking parameter information including one or more cooking parameters used for cooking by the cooking device 300, based on the above-described dish information and the target nutritional information 131a acquired by the input acquisition unit 1031. The cooking parameter information is the initial cooking parameter information 141. In the present embodiment, furthermore, the one or more cooking parameters include a cooking parameter indicating a temperature used for cooking by the cooking device 300, and a cooking parameter indicating a time used for cooking by the cooking device 300. The one or more cooking parameters further include a cooking parameter indicating a pressure used for cooking by the cooking device 300.

The parameter output unit 1037 transmits the initial cooking parameter information 141 to the cooking device 300 via the server communication unit 105. When the transmitted initial cooking parameter information 141 is acquired via the cooking communication unit 305, the cooking unit 320 of the cooking device 300 starts cooking in accordance with the initial cooking parameter information 141.

The nutritional information acquisition unit 1033 acquires the nutritional information 131 using, for example, the cooking state information 121 stored in the second storage unit 120 and the learning model set 150a stored in the model storage unit 150. The nutritional information 131 acquired in this way indicates one or more numerical values related to the nutrition of the item to be cooked when the cooking by the cooking device 300 is being performed, and is also referred to as cooking-in-progress nutritional information (i.e., the first nutritional information 131b or the second nutritional information 131c). That is, the nutritional information acquisition unit 1033 acquires cooking-in-progress nutritional information indicating one or more numerical values related to the nutrition of the item to be cooked when the item to be cooked is being cooked by the cooking device 300 in accordance with the cooking parameter information.

The parameter modification unit 1034 changes the initial cooking parameter information 141 to the modified cooking parameter information 142 including one or more modified cooking parameters, based on the target nutritional information 131a and the cooking-in-progress nutritional information described above. In other words, the parameter modification unit 1034 modifies the initial cooking parameter information 141 to the modified cooking parameter information 142. The modification of the initial cooking parameter information 141 is performed using the learning model set 150a stored in the model storage unit 150.

The parameter output unit 1037 outputs a control signal including the modified cooking parameter information 142 to the cooking device 300 via the server communication unit 105. In other words, the parameter output unit 1037 transmits a control signal including the modified cooking parameter information 142. When the transmitted modified cooking parameter information 142 is acquired via the cooking communication unit 305, the cooking unit 320 of the cooking device 300 stops the cooking according to the initial cooking parameter information 141, and performs cooking in accordance with the modified cooking parameter information 142.

Also in the transmission of the initial cooking parameter information 141 described above, the parameter output unit 1037 may transmit a control signal including the initial cooking parameter information 141 to the cooking device 300 in a manner similar to that of the modified cooking parameter information 142. In the following, the initial cooking parameter information 141 or the modified cooking parameter information 142 is transmitted or output in a state in which such information is included in the control signal.

The processing unit 1035 executes processes different from the processes executed by the components included in the server control unit 103 other than the processing unit 1035. For example, the processing unit 1035 transmits the third nutritional information 131d stored in the third storage unit 130 to the operation terminal 200 via the server communication unit 105.

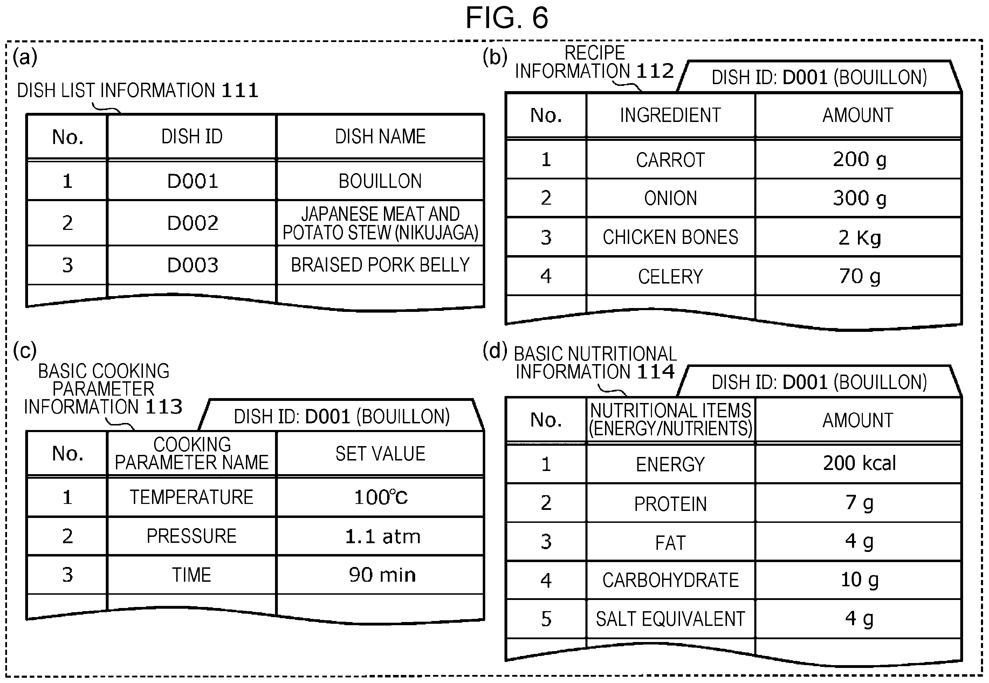

Fig. 6 is a diagram illustrating an example of the dish list information 111, the recipe information 112, the basic cooking parameter information 113, and the basic nutritional information 114.

For example, as illustrated in Fig. 6(a), the dish list information 111 indicates, for each record number, a dish ID and a dish name corresponding to the record number. The dish ID is identification information for identifying the dish name and the dish. Specifically, in the record number "1", the dish ID "D001" and the dish name "bouillon" are indicated in association with each other.

For example, as illustrated in Fig. 6(b), the recipe information 112 is associated with a dish ID and indicates one or more ingredients used in the dish identified by the dish ID and the amount of each of the one or more ingredients. Specifically, the recipe information 112 indicates, for each record number, ingredients corresponding to the record number and the amounts of the ingredients. More specifically, in the recipe information 112 associated with the dish ID "D0001", i.e., the dish name "bouillon", for the record number "1", the ingredient "carrot" and the amount "200 g" of the ingredient "carrot" are indicated in association with each other.

For example as illustrated in Fig. 6(c), the basic cooking parameter information 113 is associated with a dish ID and indicates one or more cooking parameters used by the cooking device 300 to prepare the dish identified by the dish ID. Each of the one or more cooking parameters is composed of a cooking parameter name and a set value. Specifically, the basic cooking parameter information 113 indicates, for each record number, a cooking parameter name and a set value corresponding to the record number. More specifically, in the basic cooking parameter information 113 associated with the dish ID "D0001", i.e., the dish name "bouillon", for the record number "1", the cooking parameter name "temperature" and the set value "100°C" are indicated in association with each other. That is, the record number "1" indicates the temperature "100°C" as a cooking parameter. The set value may be indicated as a numerical range such as "90°C to 110°C", for example. In a case where one numerical value, such as "100°C", is indicated as the set value, the numerical value may be an average or median of set values (e.g., temperatures) from the start of cooking to the end of cooking by the cooking device 300.

For example, as illustrated in Fig. 6(d), the basic nutritional information 114 is associated with a dish ID and indicates one or more numerical values related to the basic nutrition of the dish indicated by the dish ID. The one or more numerical values related to the nutrition specifically indicate energy and the amount of each of one or more nutrients. Specifically, the basic nutritional information 114 indicates, for each record number, a nutritional item and an amount corresponding to the record number. More specifically, in the basic nutritional information 114 associated with the dish ID "D0001", i.e., the dish name "bouillon", for the record number "1", the nutritional item "energy" and the amount "200 kcal" are indicated in association with each other. That is, the record number "1" indicates "200 kcal" of energy as a numerical value related to the nutrition. The amount may be indicated as a numerical range such as "180 kcal to 220 kcal", for example.

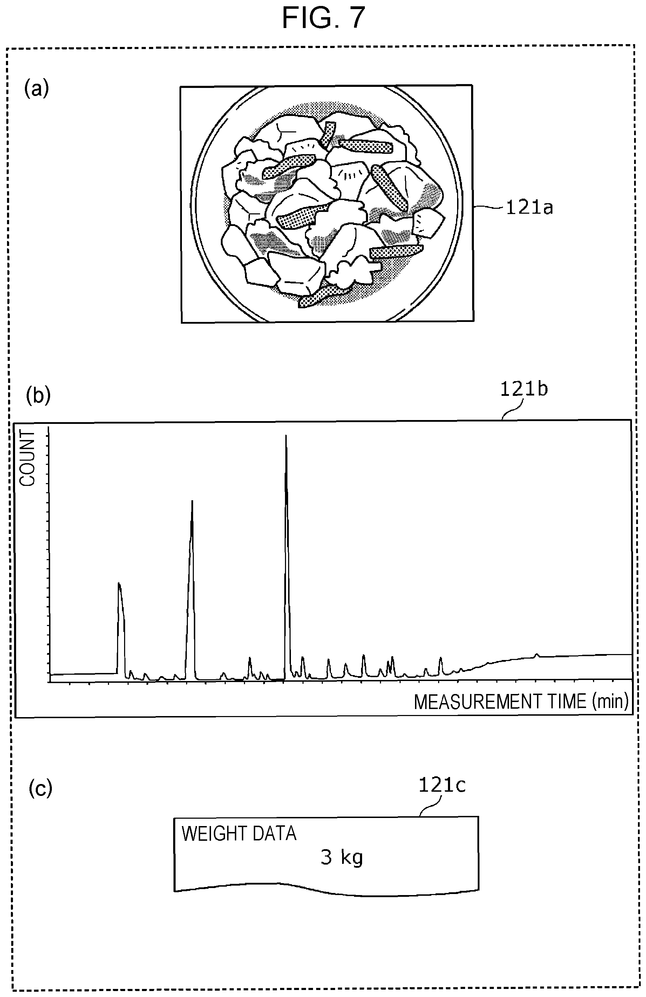

Fig. 7 is a diagram illustrating an example of image data, chemical analysis data, and weight data included in the cooking state information 121.

For example, as illustrated in Fig. 7(a), image data 121a is data indicating an image of an item to be cooked placed in the cooking device 300. The image data 121a is obtained by capturing an image using the image capturing unit 312 of the cooking device 300.

For example, as illustrated in Fig. 7(b), chemical analysis data 121b is data indicating a graph obtained by liquid chromatography, for example. The horizontal axis of the graph indicates the measurement time, and the vertical axis of the graph indicates the count number of a component measured at the measurement time. The count number may be interpreted as an intensity. The chemical analysis data 121b is obtained by analysis using the chemical analysis unit 313 of the cooking device 300. The illustrated graph could be considered to indicate the amount of each of one or more chemical components. The graph may be referred to as a chromatogram. The chemical analysis unit 313 may acquire data directly indicating the respective amounts of several chemical components as the chemical analysis data 121b, rather than data indicating the graph itself. That is, the chemical analysis unit 313 may derive the respective amounts of predetermined chemical components from the graph, and output the chemical analysis data 121b directly indicating the derived amounts of the chemical components.

For example, as illustrated in Fig. 7(c), weight data 121c is data indicating the weight of the item to be cooked placed in the cooking device 300. The weight data 121c is obtained by measurement using the weight measurement unit 311 of the cooking device 300.

### [Screen Display]

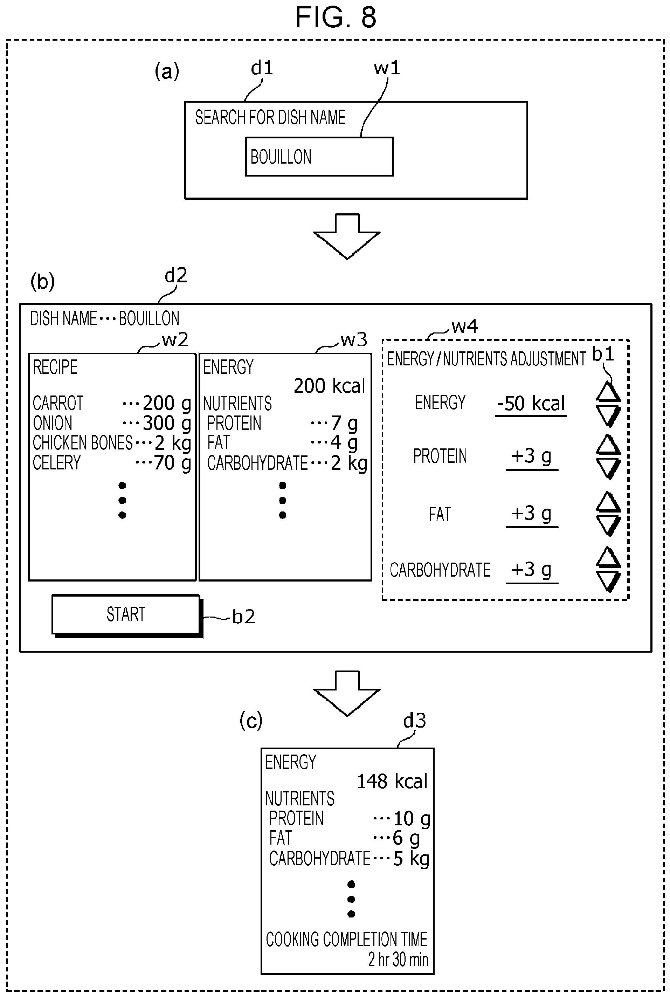

Fig. 8 is a diagram illustrating an example of screens displayed on the operation terminal 200.

For example, as illustrated in Fig. 8(a), the terminal control unit 203 of the operation terminal 200 displays a search screen d1 on the display unit 202. The search screen d1 has an input field w1 for receiving input of a dish name. The cook performs an input operation on the input unit 201 of the operation terminal 200 to fill in the input field w1 with the dish name of a dish that the cook is to prepare. For example, the cook fills in the input field w1 with the dish name "bouillon". As a result, the terminal control unit 203 acquires input information indicating the dish name "bouillon", and stores the input information in the terminal storage unit 204. The terminal control unit 203 also transmits the input information to the server 100 via the terminal communication unit 205.

The input acquisition unit 1031 of the server 100 acquires the input information from the operation terminal 200 via the server communication unit 105 as dish information, and stores the dish information in the server storage unit 104, for example. The processing unit 1035 searches the dish list information 111 stored in the first storage unit 110 for the dish name "bouillon" indicated by the dish information acquired by the input acquisition unit 1031. When the dish name "bouillon" is found from the dish list information 111, the processing unit 1035 identifies the dish ID associated with the dish name "bouillon". Then, the processing unit 1035 acquires the recipe information 112 and the basic nutritional information 114 associated with the dish ID from the first storage unit 110.

The processing unit 1035 uses the recipe information 112 and the basic nutritional information 114 to generate, for example, a nutrition input screen d2 illustrated in Fig. 8(b), and transmits information indicating the nutrition input screen d2 to the operation terminal 200 via the server communication unit 105. When the information is acquired from the server 100 via the terminal communication unit 205, the terminal control unit 203 of the operation terminal 200 displays the nutrition input screen d2 indicated by the information on the display unit 202. The nutrition input screen d2 includes a recipe field w2, a basic nutrition field w3, a nutrition adjustment field w4, and a start button b2. The recipe field w2 displays one or more ingredients for preparing the dish "bouillon" and the amount of each of the one or more ingredients, which are indicated in the recipe information 112. The basic nutrition field w3 displays the energy of the dish "bouillon" and the respective amounts of the nutrients contained in the dish "bouillon", which are indicated in the basic nutritional information 114.

The nutrition adjustment field w4 displays operation buttons b1 for changing the energy and the respective amounts of the nutrients displayed in the basic nutrition field w3. The cook operates, among the operation buttons b1, the operation button b1 corresponding to the energy or the nutrient that the cook wishes to change. When input information corresponding to an input operation for the operation button b1 is received, the terminal control unit 203 of the operation terminal 200 changes the energy or the amount of the nutrient corresponding to the operation button b1.

For example, when the operation button b1 corresponding to the energy is operated, the terminal control unit 203 changes the energy "200 kcal" displayed in the basic nutrition field w3 by "-50 kcal". That is, the terminal control unit 203 receives the energy "150 kcal" as the energy desired by the cook or as the target energy. Likewise, when the operation button b1 corresponding to the nutrient "protein" is operated, the terminal control unit 203 changes the amount "7 g" of protein displayed in the basic nutrition field w3 by "+3 g". That is, the terminal control unit 203 receives the amount "10 g" of protein as the amount of protein desired by the cook or as the target amount of protein. If the cook wishes to change the amount of any nutrient other than the nutrient "protein", the cook repeatedly operates the operation button b1 corresponding to that nutrient. Then, the cook operates the start button b2 displayed on the nutrition input screen d2. When an input operation for the start button b2 is received, the terminal control unit 203 acquires input information indicating targets for the energy and the respective amounts of the nutrients "protein", "fat", and "carbohydrate" as the target nutritional information 131a. Then, the terminal control unit 203 transmits the target nutritional information 131a to the server 100 via the terminal communication unit 205.

The range of change in the energy and the range of change in amounts of the nutrients may be limited within a predetermined range. Furthermore, when one adjustment item among the energy and the amounts of the nutrients is changed, the remaining adjustment items may also be changed in conjunction with the change of the one adjustment item. In addition, in the nutrition adjustment field w4, not all of the energy and the amounts of the nutrients indicated in the basic nutrition field w3 may be displayed, and only one of them may be displayed as a changeable adjustment item.

The nutritional information acquisition unit 1033 of the server 100 acquires the target nutritional information 131a from the operation terminal 200 via the server communication unit 105, and stores the target nutritional information 131a in the third storage unit 130. Then, the server control unit 103 derives the initial cooking parameter information 141 using the target nutritional information 131a. Further, the server control unit 103 modifies the initial cooking parameter information 141 to the modified cooking parameter information 142 by using the cooking state information 121 acquired by the cooking device 300 or the like. Further, the server control unit 103 estimates the energy of the dish "bouillon" obtained by cooking according to the initial cooking parameter information 141 and the modified cooking parameter information 142 by the cooking device 300, and the respective amounts of the nutrients contained in the dish "bouillon". That is, the server control unit 103 derives the third nutritional information 131d as the final nutritional information. Then, the server control unit 103 stores the third nutritional information 131d in the third storage unit 130, and further transmits the third nutritional information 131d to the operation terminal 200 via the server communication unit 105. In addition, the server control unit 103 may transmit the modified cooking parameter of time included in the modified cooking parameter information 142 described above to the operation terminal 200 via the server communication unit 105.

The terminal control unit 203 of the operation terminal 200 acquires the third nutritional information 131d and the modified cooking parameter of time from the server 100 via the terminal communication unit 205. Then, for example, as illustrated in Fig. 8(c), the terminal control unit 203 displays a final nutrition screen d3 for displaying the third nutritional information 131d and the like on the display unit 202. The final nutrition screen d3 displays the energy and the respective amounts of the nutrients, which are indicated by the third nutritional information 131d. The final nutrition screen d3 further displays the time indicated by the modified cooking parameter described above as, for example, the cooking completion time "2 hr 30 min".

By looking at the energy and the amounts of the nutrients displayed on the final nutrition screen d3 in response to an input of the target nutritional information 131a, the cook can recognize that a dish containing the energy and the amounts of the nutrients according to the target nutritional information 131a will be prepared. Alternatively, even when an attempt is made to cook a dish so that the energy and the amounts of the nutrients of the dish are in accordance with the target nutritional information 131a, the cook can recognize that a dish containing energy different from that of the target nutritional information 131a or a dish containing nutrients in amounts different from those of the target nutritional information 131a will be prepared. Alternatively, the cook can check how close the energy and the like of a finally prepared dish are to the desired ones. The cook can also grasp when cooking of such a dish will be completed.

When the energy or the amounts of the nutrients are displayed as a numerical range in the basic nutrition field w3, for example, the median of the numerical range may be changed by using the corresponding operation button b1 in the nutrition adjustment field w4. In this case, the width from the minimum value to the maximum value of the numerical range may be maintained constant. For example, the energy "180 kcal to 220 kcal" is displayed in the basic nutrition field w3, and is changed by "-50 kcal" through the operation of the operation button b1. In this case, the terminal control unit 203 receives the energy "130 kcal to 170 kcal" as the energy desired by the cook or as the target energy. In addition, in the initial cooking parameter information 141 and the modified cooking parameter information 142, for example, the median of the numerical range of the energy "180 kcal to 220 kcal" may be changed to approach "130 kcal to 170 kcal".

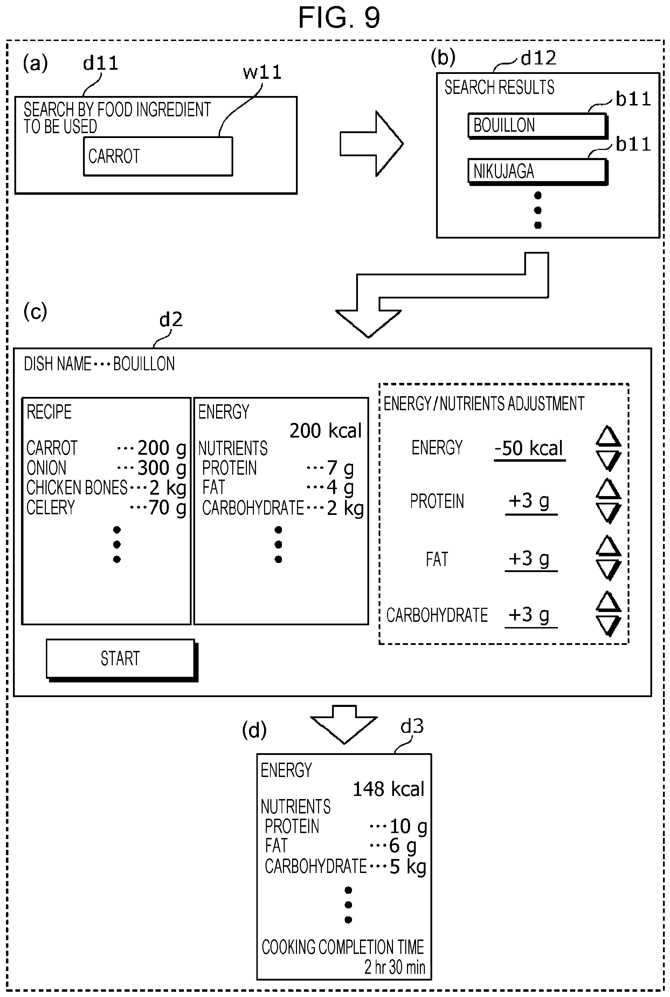

Fig. 9 is a diagram illustrating another example of screens displayed on the operation terminal 200.

In the example illustrated in Fig. 8, the cook directly inputs a dish name. Alternatively, the cook may input the name of an ingredient (i.e., food ingredient) used in the dish with the dish name. For example, as illustrated in Fig. 9(a), the terminal control unit 203 of the operation terminal 200 displays a food ingredient input screen d11 on the display unit 202. The food ingredient input screen d11 has an input field w11 for receiving input of the name of a food ingredient that the cook wishes to use. The cook performs an input operation on the input unit 201 of the operation terminal 200 to fill in the input field w11 with the name of a food ingredient that the cook wishes to use. For example, the cook fills in the input field w11 with the name of the food ingredient "carrot". As a result, the terminal control unit 203 acquires input information indicating the name of the food ingredient "carrot", and stores the input information in the terminal storage unit 204. The terminal control unit 203 also transmits the input information to the server 100 via the terminal communication unit 205.

The input acquisition unit 1031 of the server 100 acquires the input information from the operation terminal 200 via the server communication unit 105, and stores the input information in the server storage unit 104, for example. The input acquisition unit 1031 searches the pieces of recipe information 112 stored in the first storage unit 110 for one or more pieces of recipe information 112 that indicate the name of the food ingredient "carrot". Then, the input acquisition unit 1031 identifies the dish ID associated with each of the one or more pieces of recipe information 112. That is, the input acquisition unit 1031 identifies the dish name identified by the dish ID. The input acquisition unit 1031 transmits search result information indicating the identified one or more dish names to the operation terminal 200 via the server communication unit 105.

When the search result information is acquired from the server 100 via the terminal communication unit 205, for example, as illustrated in Fig. 9(b), the terminal control unit 203 of the operation terminal 200 displays a search result screen d12 on the display unit 202. The search result screen d12 includes, for each of the one or more dishes indicated by the search result information, a dish name button b11 on which the dish name of the dish is written. The cook performs an input operation on the input unit 201 of the operation terminal 200 to select the dish name button b11 corresponding to a dish that the cook wishes to prepare. For example, the cook selects the dish name button b11 on which the dish name "bouillon" is written. As a result, the terminal control unit 203 of the operation terminal 200 acquires input information indicating the dish name "bouillon" written on the selected dish name button b11, and stores the input information in the terminal storage unit 204. The terminal control unit 203 also transmits the input information to the server 100 via the terminal communication unit 205.

The input acquisition unit 1031 of the server 100 acquires the input information from the operation terminal 200 via the server communication unit 105 as dish information, and stores the dish information in the server storage unit 104, for example. The processing unit 1035 searches the dish list information 111 stored in the first storage unit 110 for the dish name "bouillon" indicated by the dish information acquired by the input acquisition unit 1031. When the dish name "bouillon" is found from the dish list information 111, the processing unit 1035 identifies the dish ID associated with the dish name "bouillon". Then, the processing unit 1035 acquires the recipe information 112 and the basic nutritional information 114 associated with the dish ID from the first storage unit 110.

Thereafter, the operation terminal 200 communicates with the server 100 to display the nutrition input screen d2 and the final nutrition screen d3 on the display unit 202, as illustrated in Figs. 9(c) and (d), like Figs. 8(b) and (c).

The operation terminal 200, as described above, is a computer that performs an information providing method for providing information related to the cooking device 300 that cooks an item to be cooked. That is, in response to an operation performed by a user who is a cook, the operation terminal 200 receives (a) dish information indicating a dish obtained by cooking an item to be cooked, and (b) the target nutritional information 131a indicating a target for one or more numerical values related to nutrition. Then, the operation terminal 200 outputs final nutritional information indicating one or more numerical values related to the nutrition of the dish, which is derived based on the dish information and the target nutritional information 131a and based on cooking-in-progress nutritional information that is the nutritional information 131 of the item to be cooked when the item to be cooked is being cooked by the cooking device 300. The final nutritional information is the third nutritional information 131d described above. The one or more numerical values related to the nutrition are at least one of the energy and the amount of each of one or more nutrients.

Accordingly, when dish information indicating a dish desired by a cook and the target nutritional information 131a desired by the cook are received, one or more numerical values (i.e., energy and the like) related to the nutrition of a finally prepared dish in accordance with the received information can be presented to the cook. This allows the cook to appropriately grasp the nutrition of the dish obtained by cooking. That is, the cook can check whether a dish containing the energy and the like desired by the cook will be prepared, or how close the energy of a finally prepared dish is to the desired one.

In the example described above, the input unit 201 of the operation terminal 200 receives text data such as a dish name or the names of food ingredients through an input operation performed by the cook. Alternatively, the input unit 201 may receive a voice signal. In this case, the input unit 201 includes a microphone, and receives a voice signal output from the microphone. Then, the terminal control unit 203 performs voice recognition on the voice signal to obtain input information indicating a dish name or the names of food ingredients.

### [Processing Operation]

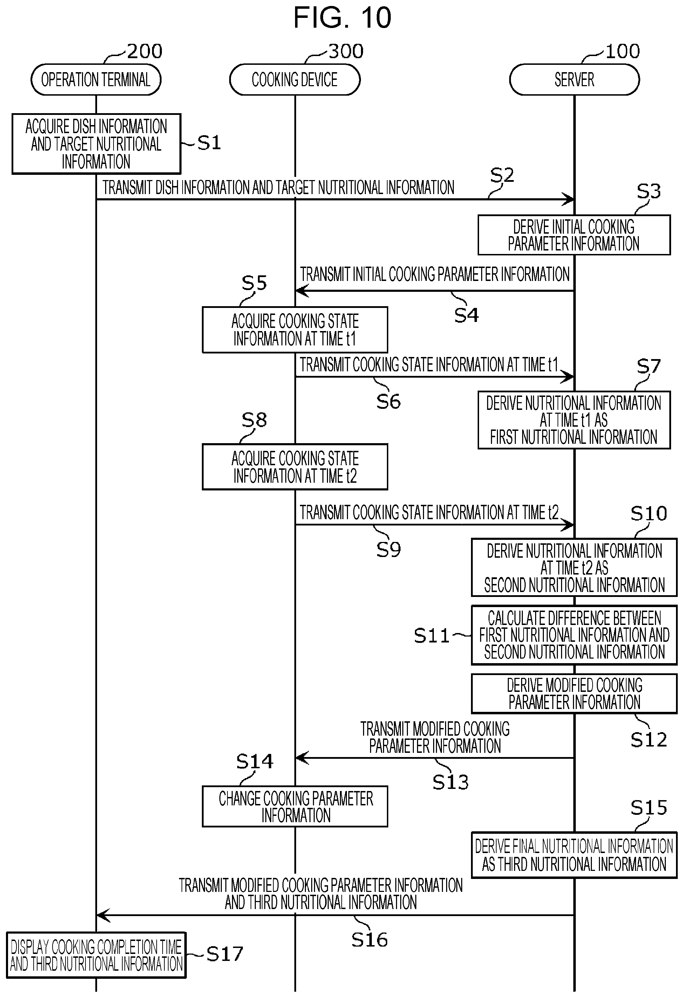

Fig. 10 is a sequence diagram illustrating an example of a processing operation of the information processing system 1000.

### (Step S1)

First, the operation terminal 200 acquires dish information and the target nutritional information 131a in accordance with an input operation performed on the input unit 201 by the cook.

### (Step S2)

The operation terminal 200 transmits the dish information and the target nutritional information 131a acquired in step S1 to the server 100.

### (Step S3)

When the dish information and the target nutritional information 131a transmitted from the operation terminal 200 in step S2 are received, the server 100 derives the initial cooking parameter information 141 using the received information. The initial cooking parameter information 141 is derived using the learning model set 150a corresponding to the dish indicated by the dish information described above among the learning model sets 150a stored in the model storage unit 150. Specifically, the first learning model 151 included in the learning model set 150a is used.

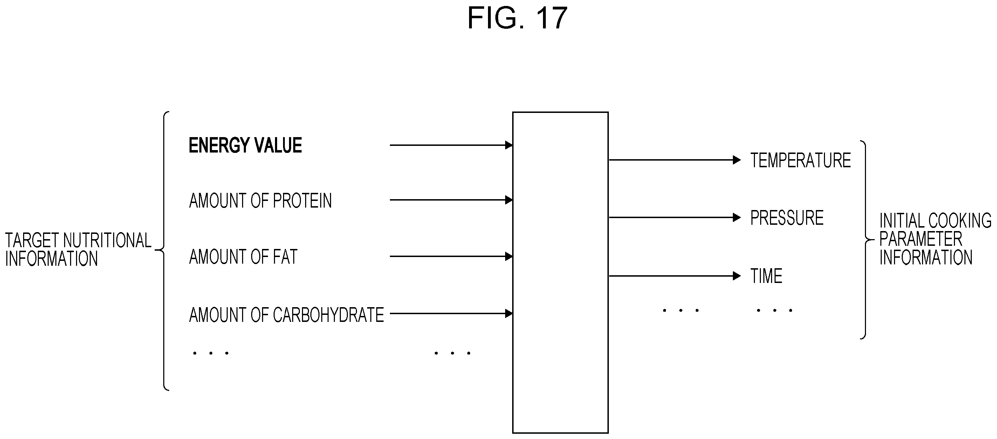

Fig. 17 illustrates an example of the first learning model 151. The input to the first learning model 151 is the target nutritional information 131a. The output from the first learning model 151 is the initial cooking parameter information 141.

The target nutritional information 131a includes an energy value of the dish indicated by the dish information, an amount of protein of the dish indicated by the dish information, an amount of fat of the dish indicated by the dish information, and an amount of carbohydrate of the dish indicated by the dish information. The energy value, the amount of protein, the amount of fat, and the amount of carbohydrate are specified by the user.

The initial cooking parameter information 141 includes the temperature, pressure, and time at which the cooking device cooks an item to be cooked such that the dish indicated by the dish information has the energy value, the amount of protein, the amount of fat, the amount of carbohydrate, etc. specified by the user. The item to be cooked may include one or more ingredients.

### (Step S4)

The server 100 transmits the derived initial cooking parameter information 141 to the cooking device 300.

### (Step S5)

When the initial cooking parameter information 141 is received from the server 100, the cooking device 300 controls the pressure, the temperature, and the time in accordance with the initial cooking parameter information 141 to start cooking the item to be cooked placed in the cooking device 300. Then, the cooking device 300 acquires the cooking state information 121 at time t1. The time t1 is a time at which a first time period has elapsed since the start of cooking by the cooking device 300. The first time period may be a predetermined time period, or may be 0 hours. That is, the time t1 may be a time at which the cooking device 300 starts cooking.

### (Step S6)

The cooking device 300 transmits the cooking state information 121 at the time t1 to the server 100.

### (Step S7)

When the cooking state information 121 at the time t1 is received from the cooking device 300, the server 100 uses the cooking state information 121 to derive the nutritional information 131 of the item to be cooked at the time t1 as the first nutritional information 131b. The first nutritional information 131b is cooking-in-progress nutritional information. The first nutritional information 131b is derived using the learning model set 150a corresponding to the dish indicated by the dish information described above among the learning model sets 150a stored in the model storage unit 150. Specifically, the second learning model 152 included in the learning model set 150a is used.

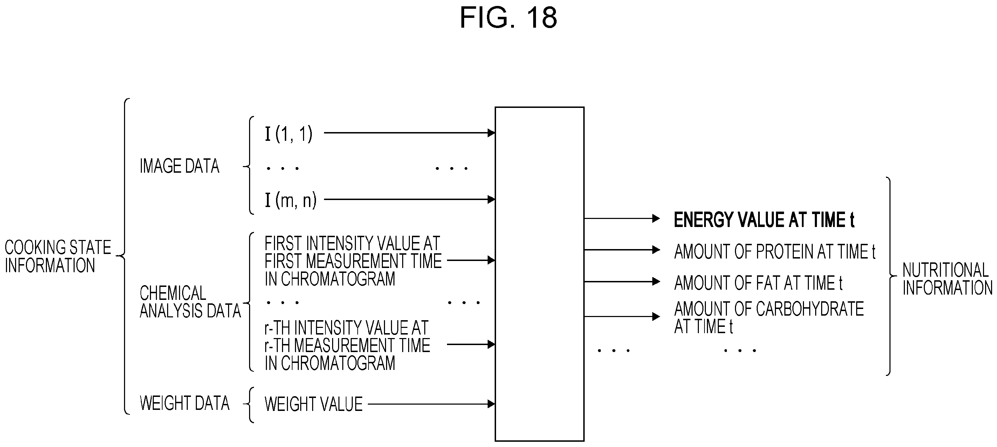

Fig. 18 illustrates an example of the second learning model 152. The input to the second learning model 152 is the cooking state information 121. The output from the second learning model 152 is the nutritional information 131.

The cooking state information 121 includes image data 121a, chemical analysis data 121b, and gravity data 121c.

The image data 121a may be m × n pixel values of an image (see, for example, Fig. 7(a)), captured by the image capturing unit 312 at time t, of the item to be cooked that is cooked by the cooking device 300 controlled based on the initial cooking parameter information 141 from the cooking start time to the time t. The m × n pixel values may be I(1, 1), ..., and I(m, n). The value I(1, 1) is the pixel value of a pixel (1, 1) of the image, ... and, the value I(m, n) is the pixel value of a pixel (m, n) of the image. The pixel (1, 1) is located at (x, y) = (1, 1) in the image, ..., and the pixel (m, n) is located at (x, y) = (m, n) in the image. Here, m may be an integer greater than or equal to 2, and n may be an integer greater than or equal to 2. An example of the XY coordinate axes is illustrated in Fig. 7(a).

The chemical analysis data 121b may be r intensity values in a chromatogram (see, for example, Fig. 7(b)) obtained by the chemical analysis unit 313 starting, at the time t, measurement of the item to be cooked that is cooked by the cooking device 300 controlled based on the initial cooking parameter information 141 from the cooking start time to the time t. The r intensity values may be a first intensity value at a first measurement time in the chromatogram, ..., and an r-th intensity value at an r-th measurement time in the chromatogram. Here, r may be an integer greater than or equal to 1.

The chemical analysis data 121b may be s amounts of s chemical components. The s amounts of the s chemical components may be a first amount of a first chemical component, ..., and an s-th amount of an s-th chemical component. Here, s may be an integer greater than or equal to 1.

The weight data 121c may be a weight value, measured by the weight measurement unit 311 at the time t, of the item to be cooked that is cooked by the cooking device 300 controlled based on the initial cooking parameter information 141 from the cooking start time to the time t.

The nutritional information 131 includes the energy value, the amount of protein, the amount of fat, and the amount of carbohydrate, at the time t, of the item to be cooked that is cooked by the cooking device 300 controlled based on the initial cooking parameter information 141 from the cooking start time to the time t.

The time t described above corresponds to the time t1 in step S7. The time t described above corresponds to time t2 in step S10 described below.

### (Step S8)

The cooking device 300 acquires the cooking state information 121 at the time t2. The time t2 is a time at a time point when a second time period elapses after the first time period has elapsed since the start of cooking by the cooking device 300. The second time period may be a time period determined in advance to be longer than the first time period.

### (Step S9)

The cooking device 300 transmits the cooking state information 121 at the time t2 to the server 100.

### (Step S10)

When the cooking state information 121 at the time t2 is received from the cooking device 300, the server 100 uses the cooking state information 121 to derive the nutritional information 131 of the item to be cooked at the time t2 as the second nutritional information 131c. The second nutritional information 131c is cooking-in-progress nutritional information, like the first nutritional information 131b. The second nutritional information 131c is derived using the learning model set 150a corresponding to the dish indicated by the dish information described above among the learning model sets 150a stored in the model storage unit 150. Specifically, the second learning model 152 included in the learning model set 150a is used.

### (Step S11)

The server 100 calculates a difference between the first nutritional information 131b and the second nutritional information 131c. That is, the server 100 calculates a difference between the energy indicated in the first nutritional information 131b and the energy indicated in the second nutritional information 131c. The server 100 further calculates, for each of one or more nutrients, a difference between the amount of the nutrient indicated in the first nutritional information 131b and the amount of the nutrient indicated in the second nutritional information 131c.

The phrase "the server 100 calculates a difference between the first nutritional information 131b and the second nutritional information 131c" described above may be interpreted as "the server 100 calculates difference information based on the first nutritional information 131b and the second nutritional information 131c".

The difference information described above may include: {(energy included in the second nutritional information 131c) - (energy included in the first nutritional information 131b)}; {(amount of protein included in the second nutritional information 131c) - (amount of protein included in the first nutritional information 131b)}; {(amount of fat included in the second nutritional information 131c) - (amount of fat included in the first nutritional information 13 1b)}; and {(amount of carbohydrate included in the second nutritional information 131c) - (amount of carbohydrate included in the first nutritional information 131b)}.

The difference information described above may include:
{(energy value, at the time t2, of the item to be cooked that is cooked by the cooking device 300 controlled based on the initial cooking parameter information 141 from the cooking start time to the time t2) - (energy value, at the time t1, of the item to be cooked that is cooked by the cooking device 300 controlled based on the initial cooking parameter information 141 from the cooking start time to the time t2)};
{(amount of protein, at the time t2, of the item to be cooked that is cooked by the cooking device 300 controlled based on the initial cooking parameter information 141 from the cooking start time to the time t2) - (amount of protein, at the time t1, of the item to be cooked that is cooked by the cooking device 300 controlled based on the initial cooking parameter information 141 from the cooking start time to the time t2)};
{(amount of fat, at the time t2, of the item to be cooked that is cooked by the cooking device 300 controlled based on the initial cooking parameter information 141 from the cooking start time to the time t2) - (amount of fat, at the time t1, of the item to be cooked that is cooked by the cooking device 300 controlled based on the initial cooking parameter information 141 from the cooking start time to the time t2)}; and
{(amount of carbohydrate, at the time t2, of the item to be cooked that is cooked by the cooking device 300 controlled based on the initial cooking parameter information 141 from the cooking start time to the time t2) - (amount of carbohydrate, at the time t1, of the item to be cooked that is cooked by the cooking device 300 controlled based on the initial cooking parameter information 141 from the cooking start time to the time t2)}.

### (Step S12)

Subsequently, the server 100 derives the modified cooking parameter information 142 using the difference calculated in step S11. The modified cooking parameter information 142 is derived using the learning model set 150a corresponding to the dish indicated by the dish information described above among the learning model sets 150a stored in the model storage unit 150. Specifically, the third learning model 153 included in the learning model set 150a is used.

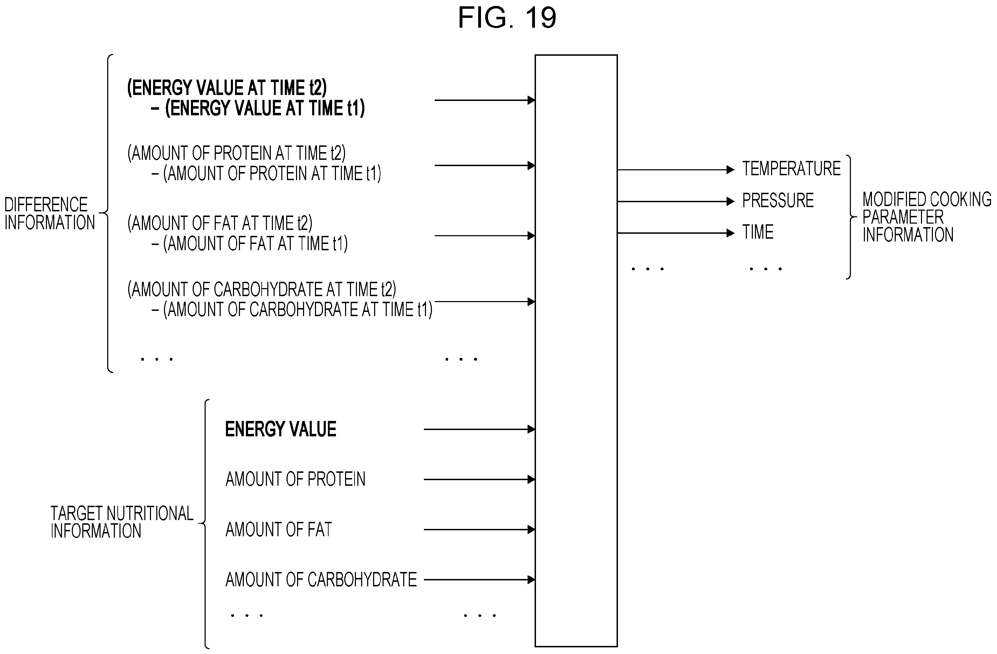

Fig. 19 illustrates an example of the third learning model 153. The inputs to the third learning model 152 are the difference information calculated based on the first nutritional information 131b and the second nutritional information 131c, and the target nutritional information 131a. The output from the third learning model 153 is the modified cooking parameter information 142.

The modified cooking parameter information 142 includes the temperature, pressure, and time at which the cooking device 300 cooks, from time t4, the item to be cooked that is cooked by the cooking device 300 controlled based on the initial cooking parameter information 141 from the cooking start time to time t3 such that the dish indicated by the dish information has the energy value, the amount of protein, the amount of fat, the amount of carbohydrate, etc. indicated in the target nutritional information 131a and specified by the user.

The time t4 may be later than the time t3, or may be equal to the time t3. The time t3 may be later than the time t1 and the time t2. The time t1 and the time t2 may be later than the cooking start time.

### (Step S13)

Then, the server 100 transmits the modified cooking parameter information 142 derived in step S12 to the cooking device 300.

### (Step S14)

The cooking device 300 receives the modified cooking parameter information 142 transmitted in step S13 and changes the initial cooking parameter information 141 to the modified cooking parameter information 142. That is, the cooking device 300 stops the cooking according to the initial cooking parameter information 141, and starts cooking according to the modified cooking parameter information 142.

### (Step S15)

Further, the server 100 uses the modified cooking parameter information 142 derived in step S12 to derive the final nutritional information 131 of the dish indicated by the dish information described above when the dish is prepared, as the third nutritional information 131d. That is, the third nutritional information 131d is estimated. The third nutritional information 131d is derived using the learning model set 150a corresponding to the dish indicated by the dish information described above among the learning model sets 150a stored in the model storage unit 150. Specifically, the fourth learning model 154 included in the learning model set 150a is used.

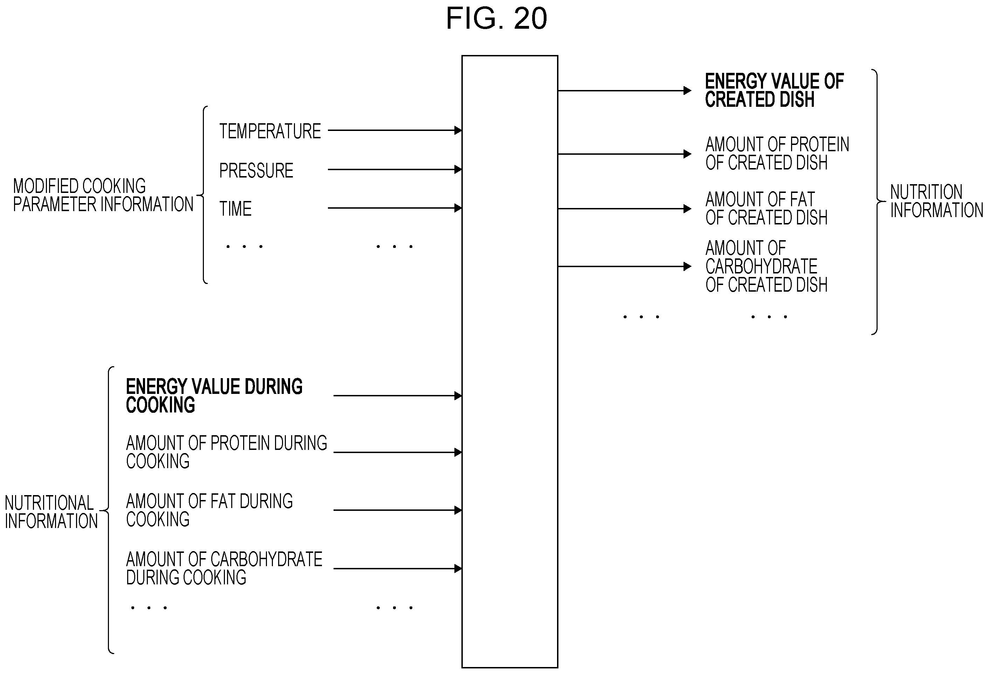

Fig. 20 illustrates an example of the fourth learning model 154. The inputs to the fourth learning model 154 are the modified cooking parameter information 142 and the nutritional content during cooking. The output from the fourth learning model 153 is the nutritional information of the created dish (i.e., the third nutritional information 131d). The third nutritional information 131d includes the energy value, the amount of protein, the amount of fat, and the amount of carbohydrate of the created dish.

### (Step S16)

The server 100 transmits to the operation terminal 200 the modified cooking parameter of time included in the modified cooking parameter information 142 derived in step S12 and the third nutritional information 131d derived in step S15. The server 100 may transmit all of the modified cooking parameters included in the modified cooking parameter information 142.

### (Step S17)

The operation terminal 200 receives the modified cooking parameter of time included in the modified cooking parameter information 142 and the third nutritional information 131d, which are transmitted in step S16. Then, as illustrated in Fig. 8(c) or Fig. 9(d), the operation terminal 200 displays the modified cooking parameter of time and the third nutritional information 131d on the display unit 202. The modified cooking parameter of time is displayed as, for example, a cooking completion time.

As described above, in the present embodiment, dish information indicating a dish desired by the cook and the target nutritional information 131a desired by the cook are acquired, based on which the initial cooking parameter information 141 is acquired. Then, when the initial cooking parameter information 141 is transmitted to the cooking device 300, cooking according to the initial cooking parameter information 141 is started by the cooking device 300. Thereafter, the initial cooking parameter information 141 used by the cooking device 300 is modified with one or more numerical values (i.e., energy and the like) related to the nutrition of the item to be cooked when cooking is being performed. Accordingly, cooking according to the modified cooking parameter information 142 makes it possible to bring the energy and the like of a finally prepared dish closer to the energy and the like indicated by the target nutritional information 131a. In other words, it is possible to bring the energy and the like of a finally prepared dish closer to the energy and the like desired by the cook. That is, even if the energy and the like desired by the cook are different from the energy and the like specified in the basic recipe, or even if the amount of the item to be cooked is not as specified in the recipe, the energy and the like of a final dish can be brought closer to the energy and the like desired by the cook. As a result, it is possible to appropriately control the nutrition of the dish without requiring the cook to adjust cooking parameters.

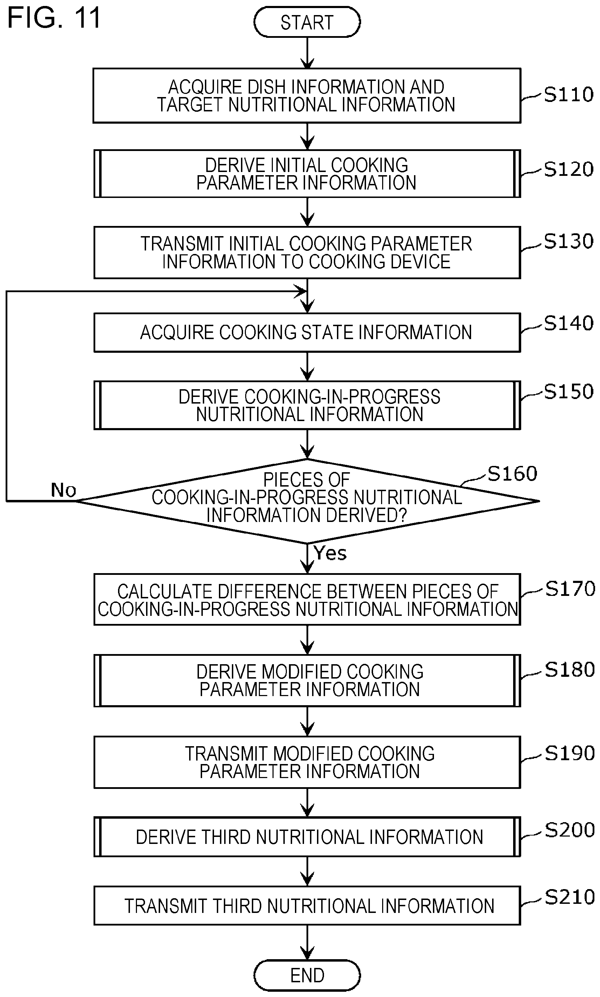

Fig. 11 is a flowchart illustrating an example of a processing operation of the server 100.

### (Step S110)

First, the input acquisition unit 1031 of the server 100 acquires dish information and the target nutritional information 131a from the operation terminal 200 via the server communication unit 105.

### (Step S120)

The parameter acquisition unit 1032 derives the initial cooking parameter information 141 using the dish information and the target nutritional information 131a.

### (Step S130)

The parameter output unit 1037 transmits the initial cooking parameter information 141 acquired in step S120 to the cooking device 300 via the server communication unit 105.

### (Step S140)

The processing unit 1035 acquires the cooking state information 121 from the cooking device 300 via the server communication unit 105, and stores the cooking state information 121 in the second storage unit 120.

### (Step S150)

The nutritional information acquisition unit 1033 derives cooking-in-progress nutritional information using the cooking state information 121 acquired in step S140.

### (Step S160)

After the processing of step S150 is performed, the parameter modification unit 1034 determines whether pieces of cooking-in-progress nutritional information have been derived. That is, the parameter modification unit 1034 determines whether the first nutritional information 131b and the second nutritional information 131c have been derived. If it is determined in the processing of step S160 that pieces of cooking-in-progress nutritional information have not been derived (No in step S160), the processing unit 1035 and the nutritional information acquisition unit 1033 repeatedly execute the processing of steps S140 and S150. That is, the nutritional information acquisition unit 1033 repeats the processing of step S150 to acquire the first nutritional information 131b and the second nutritional information 131c. Specifically, the nutritional information acquisition unit 1033 acquires, as the first nutritional information 131b, cooking-in-progress nutritional information at a time point when the first time period elapses after the start of cooking by the cooking device 300. The nutritional information acquisition unit 1033 further acquires, as the second nutritional information 131c, cooking-in-progress nutritional information at a time point when the second time period elapses after the first time period has elapsed since the start of the cooking by the cooking device 300. The time point when the first time period elapses corresponds to the time t1 in Fig. 10, and the time point when the second time period elapses corresponds to the time t2 in Fig. 10.

### (Step S170)

On the other hand, if it is determined in the processing of step S160 that pieces of cooking-in-progress nutritional information have been derived (Yes in step S160), the parameter modification unit 1034 calculates a difference between the pieces of cooking-in-progress nutritional information. That is, the parameter modification unit 1034 calculates a difference between the first nutritional information 131b and the second nutritional information 131c.

### (Step S180)

Then, the parameter modification unit 1034 derives the modified cooking parameter information 142 using the target nutritional information 131a acquired in step S110 and the difference calculated in step S170. The modified cooking parameter information 142 is information for bringing one or more numerical values related to the nutrition of a finally prepared dish closer to one or more numerical values indicated in the target nutritional information 131a. As described above, the parameter modification unit 1034 in the present embodiment changes the initial cooking parameter information 141 to the modified cooking parameter information 142 based on the target nutritional information 131a and the difference between the first nutritional information 131b and the second nutritional information 131c.

As described above, in the present embodiment, the initial cooking parameter information 141 is modified to the modified cooking parameter information 142 based on the amount of change in the cooking-in-progress nutritional information and the target nutritional information 131a. Accordingly, by taking into consideration the tendency of changes in energy and the like caused by the cooking according to the initial cooking parameter information 141, the energy and the like of a final dish can be effectively brought closer to the energy and the like desired by the cook.

### (Step S190)

The parameter output unit 1037 transmits the modified cooking parameter information 142 derived in step S180 to the operation terminal 200 and the cooking device 300 via the server communication unit 105. The parameter output unit 1037 may transmit only the modified cooking parameter of time included in the modified cooking parameter information 142 to the operation terminal 200.

### (Step S200)

The nutritional information acquisition unit 1033 derives the third nutritional information 131d using the modified cooking parameter information 142 derived in step S180. That is, the nutritional information acquisition unit 1033 acquires the third nutritional information 131d indicating one or more numerical values related to the nutrition of a dish obtained by cooking according to the modified cooking parameter information 142 by the cooking device 300, based on the modified cooking parameter information 142.

### (Step S210)

The parameter output unit 1037 transmits the third nutritional information 131d derived in step S200 to the operation terminal 200 via the server communication unit 105. The third nutritional information 131d is displayed on the display unit 202 of the operation terminal 200.

As described above, in the present embodiment, information indicating the energy and the like of a finally prepared dish is estimated as the third nutritional information 131d. Then, the third nutritional information 131d is presented to the cook. As a result, the cook can check whether a dish containing the energy and the like desired by the cook will be prepared, or how close the energy of a finally prepared dish is to the desired one.

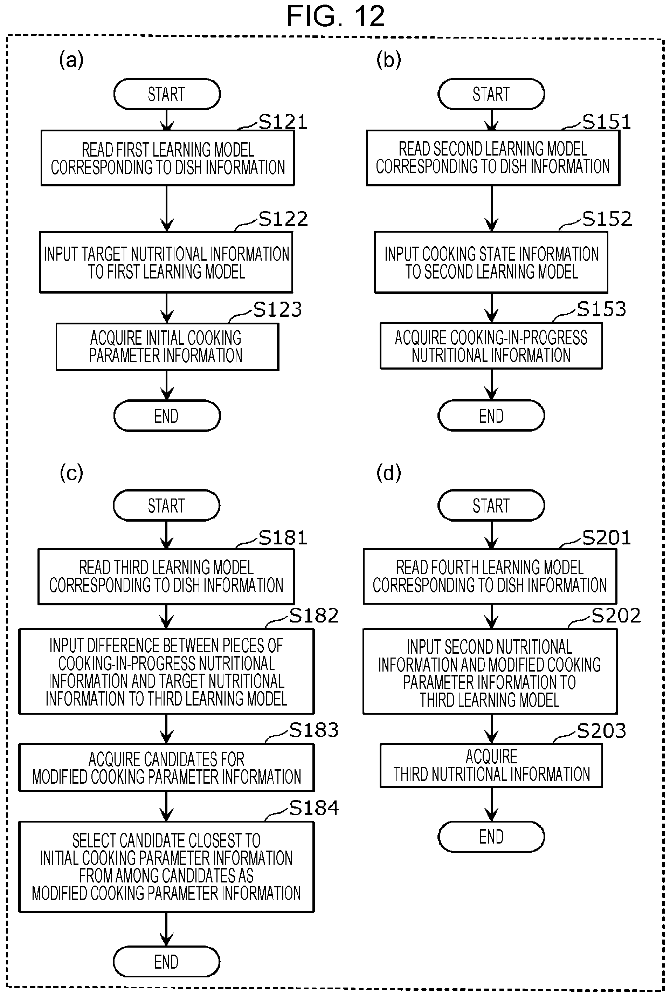

Fig. 12 illustrates flowcharts illustrating portions of the processing operation of the server 100 in detail. Specifically, Fig. 12(a) is a flowchart illustrating the detailed processing of step S120 in Fig. 11, and Fig. 12(b) is a flowchart illustrating the detailed processing of step S150 in Fig. 11. Fig. 12(c) is a flowchart illustrating the detailed processing of step S180 in Fig. 11, and Fig. 12(d) is a flowchart illustrating the detailed processing of step S200 in Fig. 11.

To derive the initial cooking parameter information 141, the parameter acquisition unit 1032 executes, for example, the processing of steps S121 to S 123 illustrated in Fig. 12(a).

### (Step S121)

The parameter acquisition unit 1032 reads the first learning model 151 corresponding to the dish information from the model storage unit 150. That is, the parameter acquisition unit 1032 reads, from the model storage unit 150, the first learning model 151 included in the learning model set 150a corresponding to the dish name indicated by the dish information.

### (Step S122)

The parameter acquisition unit 1032 inputs the target nutritional information 131a to the first learning model 151 read in step S121.

### (Step S123)

The parameter acquisition unit 1032 acquires the initial cooking parameter information 141 output from the first learning model 151 in response to the input in step S122. As a result, the initial cooking parameter information 141 is derived.

To derive cooking-in-progress nutritional information such as the first nutritional information 131b, the nutritional information acquisition unit 1033 executes, for example, the processing of steps S151 to S153 illustrated in Fig. 12(b).

### (Step S151)

The nutritional information acquisition unit 1033 reads the second learning model 152 corresponding to the dish information from the model storage unit 150. That is, the nutritional information acquisition unit 1033 reads, from the model storage unit 150, the second learning model 152 included in the learning model set 150a corresponding to the dish name indicated by the dish information.

### (Step S152)

The nutritional information acquisition unit 1033 inputs the latest cooking state information 121 to the read second learning model 152.

### (Step S153)

The nutritional information acquisition unit 1033 acquires, as cooking-in-progress nutritional information, the nutritional information 131 output from the second learning model 152 in response to the input in step S152. As a result, the cooking-in-progress nutritional information is derived.

As described above, the nutritional information acquisition unit 1033 in the present embodiment acquires cooking-in-progress nutritional information by inputting to the second learning model 152 an image of an item to be cooked, the weight of the item to be cooked, and the amount of chemical components contained in the item to be cooked when the cooking by the cooking device 300 is being performed. Accordingly, the second learning model 152 is used for the acquisition of the cooking-in-progress nutritional information. Thus, cooking-in-progress nutritional information with a high degree of accuracy can be acquired.

The image of the item to be cooked, the weight of the item to be cooked, and the amounts of the chemical components contained in the item to be cooked are included in the cooking state information 121 as the image data 121a, the weight data 121c, and the chemical analysis data 121b. In the present embodiment, furthermore, the image data 121a, the weight data 121c, and the chemical analysis data 121b are input to the second learning model 152. However, only one of the image data 121a, the chemical analysis data 121b, and the weight data 121c, or only two of the image data 121a, the chemical analysis data 121b, and the weight data 121c may be input to the second learning model 152. That is, at least one of the image data 121a, the weight data 121c, and the chemical analysis data 121b may be input to the second learning model 152. Even in such cases, cooking-in-progress nutritional information with a high degree of accuracy can be acquired.

To derive the modified cooking parameter information 142, the parameter modification unit 1034 executes, for example, the processing of steps S181 to S 184 illustrated in Fig. 12(c).

### (Step S181)

The parameter modification unit 1034 reads the third learning model 153 corresponding to the dish information from the model storage unit 150. That is, the parameter modification unit 1034 reads, from the model storage unit 150, the third learning model 153 included in the learning model set 150a corresponding to the dish name indicated by the dish information.

### (Step S182)

The parameter modification unit 1034 inputs the difference between the pieces of cooking-in-progress nutritional information and the target nutritional information 131a to the read third learning model 153.

### (Step S183)

The parameter modification unit 1034 acquires candidates output from the third learning model 153 in response to the input in step S182. Each of the candidates is a candidate for the modified cooking parameter information 142.

### (Step S184)

The parameter modification unit 1034 selects, as the modified cooking parameter information 142, a candidate closest to the initial cooking parameter information 141 from among the candidates acquired in step S183. As a result, the modified cooking parameter information 142 is derived.

As described above, the parameter modification unit 1034 in the present embodiment acquires candidates for the modified cooking parameter information 142, based on the target nutritional information 131a and the cooking-in-progress nutritional information. Then, the parameter modification unit 1034 selects, from among the candidates, a candidate closest to the initial cooking parameter information 141 as the modified cooking parameter information 142. Alternatively, the parameter modification unit 1034 may select, from among the candidates, a candidate whose difference from the initial cooking parameter information 141 is less than or equal to a threshold as the modified cooking parameter information 142. For example, each of the candidates is represented as a vector including a cooking parameter of pressure, a cooking parameter of temperature, and a cooking parameter of time. Likewise, the initial cooking parameter information 141 is also represented as a vector including a cooking parameter of pressure, a cooking parameter of temperature, and a cooking parameter of time. Thus, the parameter modification unit 1034 may calculate the distance between the vector of each of the candidates and the vector of the initial cooking parameter information 141, and select, from among the candidates, a candidate whose distance is less than or equal to a threshold or is the smallest as the modified cooking parameter information 142. Alternatively, among the candidates, for example, a candidate in which one or two predetermined cooking parameters are the same as those in the initial cooking parameter information 141 and only the remaining two or one cooking parameter is different from that in the initial cooking parameter information 141 may be selected as the modified cooking parameter information 142. The one or two predetermined cooking parameters may be a cooking parameter of temperature, a cooking parameter of pressure, or the like.

As a result, even if candidates are output from the third learning model 153, a candidate that is not greatly different from the initial cooking parameter information 141 is adopted as the modified cooking parameter information 142. Thus, the processing load on the cooking device 300 caused by the modification of the initial cooking parameter information 141 can be reduced. It is also possible to suppress significant changes in the taste of the dish.

To derive the third nutritional information 131d, the nutritional information acquisition unit 1033 executes, for example, the processing of steps S201 to S203 illustrated in Fig. 12(d).

### (Step S201)

The nutritional information acquisition unit 1033 reads the fourth learning model 154 corresponding to the dish information from the model storage unit 150. That is, the nutritional information acquisition unit 1033 reads, from the model storage unit 150, the fourth learning model 154 included in the learning model set 150a corresponding to the dish name indicated by the dish information.

### (Step S202)

The nutritional information acquisition unit 1033 inputs the second nutritional information 131c and the modified cooking parameter information 142 to the read fourth learning model 154.

### (Step S203)

The nutritional information acquisition unit 1033 acquires, as the third nutritional information 131d, the nutritional information 131 output from the fourth learning model 154 in response to the input in step S202. As a result, the third nutritional information 131d is derived.

As described above, the nutritional information acquisition unit 1033 in the present embodiment inputs at least the modified cooking parameter information 142 to the fourth learning model 154 to acquire the third nutritional information 131d.

### [Examples of Constructing Learning Models]

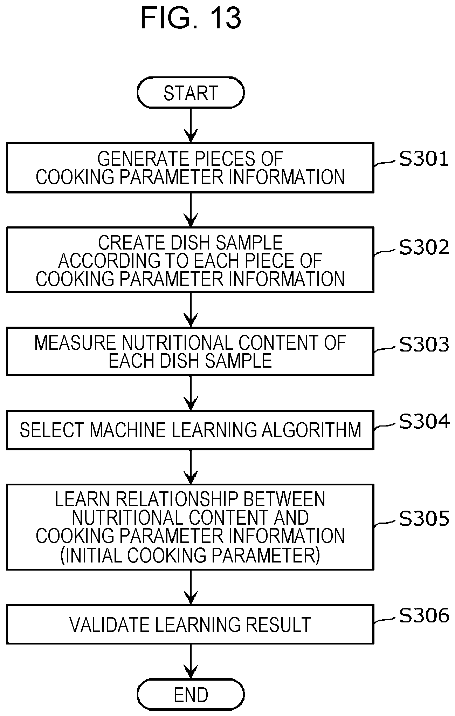

Fig. 13 is a flowchart illustrating an example of constructing the first learning model 151.

### (Step S301)

First, a model creator generates pieces of cooking parameter information for a predetermined dish. The cooking parameter information indicates one or more cooking parameters used by the cooking unit 320 of the cooking device 300.

### (Step S302)

Next, the model creator creates a sample of the dish (also referred to as a dish sample) for each of the pieces of cooking parameter information by performing cooking with the cooking device 300 according to the corresponding piece of cooking parameter information. The cooking device 300 cooks one or more food ingredients indicated by recipe information corresponding to the predetermined dish.

### (Step S303)

The model creator measures, for each of the dish samples created in step S302, the nutritional content of the dish sample. The nutritional content includes energy and amounts of nutrients contained in the dish sample.

### (Step S304)

The model creator selects a machine learning algorithm for constructing the first learning model 151 for the predetermined dish.

### (Step S305)

The model creator trains a learning model to learn the relationship between the nutritional content associated with the predetermined dish described above and the cooking parameter information in accordance with the machine learning algorithm selected in step S304.

### (Step S306)

The model creator validates the learning model trained in the processing of step S305. That is, the model creator validates whether the learning model outputs correct cooking parameter information as the initial cooking parameter information 141 in response to an input of nutritional information indicating the nutritional content to the learning model. The learning model validated to output correct cooking parameter information is stored in the model storage unit 150 of the server 100 as the first learning model 151 corresponding to the predetermined dish. The correct cooking parameter information is information based on which a dish having the nutritional content input to the learning model will be actually created by the cooking according to the cooking parameter information.

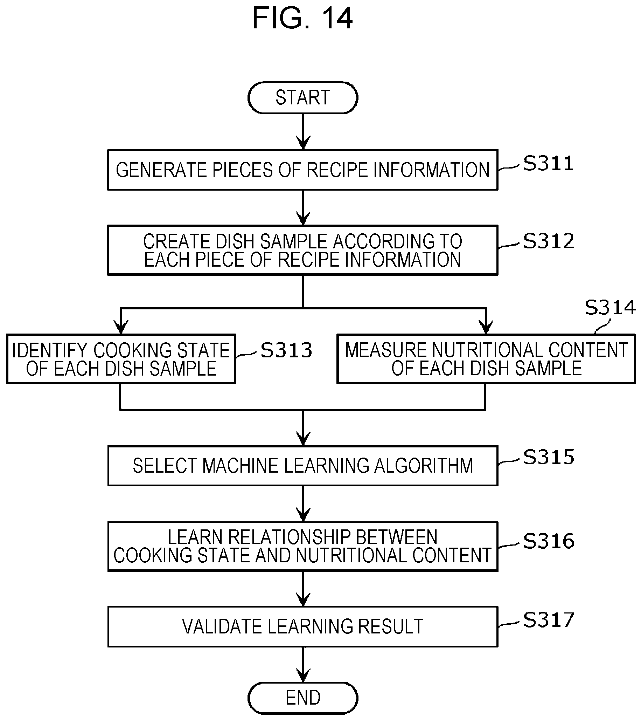

Fig. 14 is a flowchart illustrating an example of constructing the second learning model 152.

### (Step S311)

First, the model creator generates pieces of recipe information for a predetermined dish. The recipe information indicates one or more ingredients used in the predetermined dish and the amount of each of the one or more ingredients.

### (Step S312)

Next, the model creator creates a dish sample for each of the pieces of recipe information by performing cooking with the cooking device 300 according to the corresponding piece of recipe information. The dish samples may be samples at the time point when the first time period elapses or the time point when the second time period elapses, described above, after the start of cooking.

### (Step S313)

The model creator identifies, for each of the dish samples created in step S312, the cooking state of the dish sample. That is, the model creator captures an image of the dish sample using a camera to acquire image data, analyzes the dish sample using a chemical analysis device to acquire chemical analysis data, and acquires weight data of the dish sample using a weighing scale. As a result, the image data, the chemical analysis data, and the weight data are identified as the cooking state of the dish sample.

### (Step S314)

The model creator measures, for each of the dish samples created in step S312, the nutritional content of the dish sample, that is, energy and amounts of nutrients contained in the dish sample.

### (Step S315)

The model creator selects a machine learning algorithm for constructing the second learning model 152 for the predetermined dish.

### (Step S316)

The model creator trains a learning model to learn the relationship between the cooking state and the nutritional content in accordance with the machine learning algorithm selected in step S315.

### (Step S317)

The model creator validates the learning model trained in the processing of step S316. That is, the model creator validates whether the learning model outputs nutritional information indicating correct nutritional content in response to an input of cooking state information indicating the cooking state to the learning model. The learning model validated to output nutritional information indicating correct nutritional content is stored in the model storage unit 150 of the server 100 as the second learning model 152 corresponding to the predetermined dish. The correct nutritional content is the actual nutritional content of the dish sample having the cooking state input to the learning model.

As described above, the second learning model 152 in the present embodiment is trained by machine learning such that, in response to an input of an image of one or more food ingredients being cooked by the cooking device 300, the weight of the one or more food ingredients, and the amount of chemical components contained in the one or more food ingredients, one or more numerical values related to the nutrition of the one or more food ingredients are output. The one or more numerical values related to the nutrition of the one or more food ingredients correspond to the nutritional content described above or the nutritional information 131. The image, the weight, and the amount of the chemical components of the one or more food ingredients correspond to the image data 121a, the weight data 121c, and the chemical analysis data 121b described above, respectively, and these pieces of data represent the cooking state of the dish sample described above. **In** addition, in the machine learning in the present embodiment, the image, the weight, and the amount of the chemical components are input to the learning model. However, only one of the image, the weight, and the amount of the chemical components, or only two of the image, the weight, and the amount of the chemical components may be input to the learning model. That is, the second learning model 152 may be trained by machine learning such that, in response to an input of at least one of an image, a weight, and an amount of chemical components of one or more food ingredients, nutritional information indicating the nutritional content of the one or more food ingredients is output.

Accordingly, when the nutritional information acquisition unit 1033 inputs at least one of the image data 121a, the weight data 121c, and the chemical analysis data 121b of the item to be cooked to the second learning model 152, cooking-in-progress nutritional information with a high degree of accuracy can be acquired from the second learning model 152.

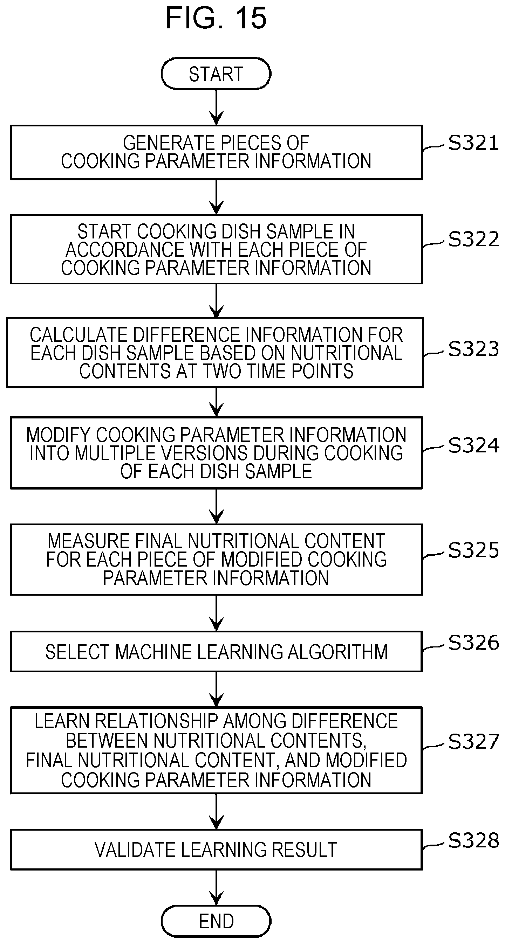

Fig. 15 is a flowchart illustrating an example of constructing the third learning model 153.

### (Step S321)

First, a model creator generates pieces of cooking parameter information for a predetermined dish.

### (Step S322)

Next, the model creator starts, for each of the pieces of cooking parameter information, cooking a dish sample using the cooking device 300 in accordance with the corresponding piece of cooking parameter information.

### (Step S323)

After cooking is started in step S322, the model creator measures, for each dish sample, the nutritional content of the dish sample at two time points. Then, the model creator calculates difference information based on the nutritional contents measured at the two time points. The two time points are time points when the cooking by the cooking device 300 is being performed. That is, the nutritional content to be measured is the nutritional content during cooking. The two time points may be the time point when the first time period elapses and the time point when the second time period elapses, described above.

### (Step S324)

Then, the model creator modifies the cooking parameter information into multiple versions during cooking. That is, the model creator stops the cooking according to the cooking parameter information obtained before the modification, and starts cooking according to the modified cooking parameter information.

### (Step S325)

The model creator measures, for each of the dish samples created in accordance with the modified cooking parameter information, the nutritional content of the dish sample, that is, energy and amounts of nutrients contained in the dish sample. The nutritional content measured at this time is the final nutritional content after cooking.

### (Step S326)

The model creator selects a machine learning algorithm for constructing the third learning model 153 for the predetermined dish.

### (Step S327)

The model creator trains a learning model to learn the relationship among the difference between the nutritional contents during cooking calculated in step S323, the final nutritional content measured in step S325, and the modified cooking parameter information in accordance with the machine learning algorithm selected in step S326.

### (Step S328)

The model creator validates the learning model trained in the processing of step S327. That is, the model creator validates whether the learning model outputs correct modified cooking parameter information as the modified cooking parameter information 142 in response to an input of the difference between the nutritional contents during cooking and nutritional information indicating the final nutritional content to the learning model. The learning model validated to output correct modified cooking parameter information is stored in the model storage unit 150 of the server 100 as the third learning model 153. The final nutritional content described above corresponds to the target nutritional information 131a. The correct modified cooking parameter information is information that is actually required to prepare a dish having the final nutritional content input to the learning model in a case where the nutritional content is changed as in the difference between the nutritional contents input to the learning model. The learning model constructed in this way, i.e., the third learning model 153, makes it possible to acquire the modified cooking parameter information 142 that brings the nutritional content during cooking closer to the target nutritional information 131a.

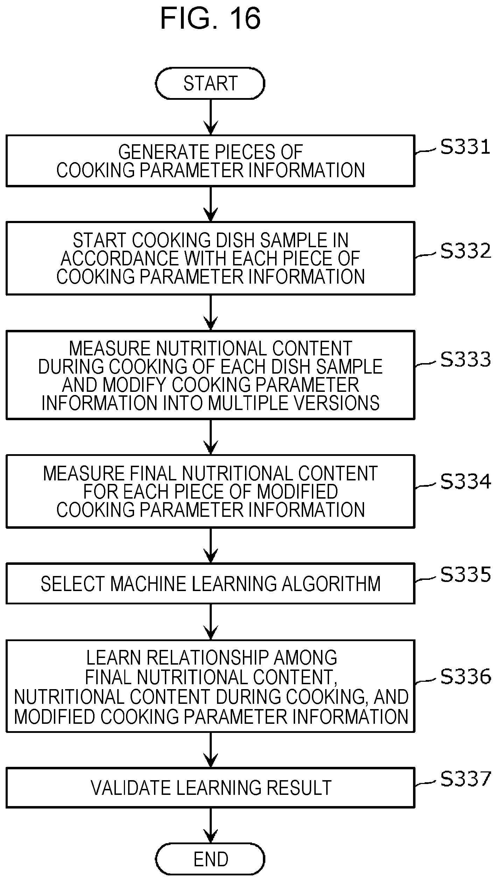

Fig. 16 is a flowchart illustrating an example of constructing the fourth learning model 154.

### (Step S331)

First, a model creator generates pieces of cooking parameter information for a predetermined dish.

### (Step S332)

Next, the model creator starts, for each of the pieces of cooking parameter information, cooking a dish sample using the cooking device 300 in accordance with the corresponding piece of cooking parameter information.

### (Step S333)

Then, after cooking is started in step S332, the model creator measures, for each dish sample, the nutritional content of the dish sample. The nutritional content measured at this time is the nutritional content during cooking. Further, the model creator modifies the cooking parameter information into multiple versions during cooking. That is, the model creator stops the cooking according to the cooking parameter information obtained before the modification, and starts cooking according to the modified cooking parameter information. The processing of step S333 may be performed at a time point when the second time period described above has elapsed since the start of cooking. The processing of step S333 may be started from a time point when the second time period described above has elapsed since the start of cooking.

### (Step S334)

The model creator measures, for each of the dish samples created in accordance with the modified cooking parameter information, the nutritional content of the dish sample, that is, energy and amounts of nutrients contained in the dish sample. The nutritional content measured at this time is the final nutritional content after cooking.

### (Step S335)

The model creator selects a machine learning algorithm for constructing the fourth learning model 154 for the predetermined dish.

### (Step S336)

The model creator trains a learning model to learn the relationship among the final nutritional content measured in step S334, the nutritional content during cooking measured in step S333, and the modified cooking parameter information in accordance with the machine learning algorithm selected in step S335.

### (Step S337)

The model creator validates the learning model trained in the processing of step S336. That is, the model creator validates whether the learning model outputs correct final nutritional content as the third nutritional information 131d in response to an input of nutritional information indicating the nutritional content during cooking and nutritional information indicating the modified cooking parameter information to the learning model. The learning model validated to output nutritional information indicating correct final nutritional content is stored in the model storage unit 150 of the server 100 as the fourth learning model 154. The correct final nutritional content is the actual nutritional content of a final dish sample obtained by cooking the dish sample having the nutritional content during cooking input to the learning model in accordance with the modified cooking parameter information input to the learning model.

As described above, the fourth learning model 154 in the present embodiment is trained by machine learning such that, at least in response to an input of one or more cooking parameters changed during the cooking by the cooking device 300, one or more numerical values related to the nutrition of a dish obtained by cooking according to the one or more cooking parameters by the cooking device 300 are output. The one or more modified cooking parameters correspond to the modified cooking parameter information 142, and the one or more numerical values related to the nutrition of the dish are information indicating the final nutritional content of the dish and correspond to the third nutritional information 131d.

Since the fourth learning model 154 trained by machine learning in the way described above is used to acquire the third nutritional information 131d, the third nutritional information 131d with a high degree of accuracy can be acquired.

As described above, the information processing system 1000 in the present embodiment can appropriately control the nutrition of a dish without requiring the cook to adjust cooking parameters. In addition, the cook can appropriately grasp the nutrition of the dish obtained by cooking. That is, the cook can check whether a dish containing the energy and the like desired by the cook will be prepared, or how close the energy of a finally prepared dish is to the desired one.

### <Other Aspects>

While an information processing system, a server, an operation terminal, and the like according to the present disclosure have been described above with reference to an embodiment, the present disclosure is not limited to this embodiment. Various modifications conceivable to a person skilled in the art may be made to the embodiment without departing from the spirit of the present disclosure, and such modifications may also be encompassed in the scope of the present disclosure.

For example, the operation terminal 200 in the embodiment described above is a device independent of the cooking device 300, but may be incorporated into the cooking device 300. That is, among the various functions of the operation terminal 200, the functions used in the information processing system 1000 may be included in the cooking device 300. Alternatively, the operation terminal 200 may be configured as a personal computer.

Furthermore, the cook in the embodiment described above may be a user who uses the information processing system 1000, or may be an operator of the operation terminal 200 or the cooking device 300.

In addition, the server 100 in the embodiment described above includes the first storage unit 110, the second storage unit 120, the third storage unit 130, the fourth storage unit 140, and the model storage unit 150, but is not required to include these recording media. The server 100 may use these recording media by, for example, communicating with a device external to the server 100 and having those recording media.

Furthermore, the server 100 in the embodiment described above acquires cooking parameter information including one or more cooking parameters used for cooking by the cooking device 300, based on the dish information and the target nutritional information 131a. That is, the server 100 acquires the initial cooking parameter information 141. However, the server 100 is not required to acquire the initial cooking parameter information 141. In this case, the server 100 acquires cooking-in-progress nutritional information of an item to be cooked when the item to be cooked is being cooked by the cooking device 300 in accordance with the basic cooking parameter information 113. Alternatively, the server 100 may acquire the initial cooking parameter information 141 generated by the cooking device 300.

In other words, the control method according to the present disclosure is a control method executed by a computer to control a cooking device that cooks an item to be cooked, and may perform the following processing operations. That is, the control method acquires dish information indicating a dish obtained by cooking the item to be cooked, acquires cooking parameter information including one or more cooking parameters used for cooking by the cooking device, based on the acquired dish information, and acquires target nutritional information indicating a target numerical value for one or more numerical values related to nutrition. The control method further acquires cooking-in-progress nutritional information indicating the one or more numerical values related to the nutrition of the item to be cooked when the item to be cooked is being cooked by the cooking device in accordance with the cooking parameter information, changes the cooking parameter information to modified cooking parameter information including one or more modified cooking parameters, based on the target nutritional information and the cooking-in-progress nutritional information, and outputs a control signal including the modified cooking parameter information. Here, the one or more numerical values related to the nutrition are a numerical value of at least one of energy and an amount of each of one or more nutrients. The cooking parameter information may be the basic cooking parameter information 113 or the initial cooking parameter information 141, and may be acquired from the cooking device 300. The control signal may be output or transmitted to the cooking device 300. Even this control method can achieve operations and effects similar to those of the control method in the embodiment described above.

In the embodiment described above, furthermore, each component may be configured by dedicated hardware or may be implemented by executing a software program suitable for each component. Each component may be implemented by a program execution unit such as a CPU or a processor reading and executing a software program recorded on a recording medium such as a hard disk or a semiconductor memory. Here, a program for implementing a system such as the server 100 and the operation terminal 200 in the embodiment described above may cause a processor to execute, for example, the steps included in the sequence diagram in Fig. 10. Further, a program for implementing the server 100 in the embodiment described above may cause a processor to execute the steps included in each of the flowcharts in Figs. 11 and 12.

### (Hardware Configuration)

The server 100 or the operation terminal 200 described above may be configured by, specifically, a computer system including a microprocessor, a ROM, a RAM, a hard disk drive, a display unit, a keyboard, a mouse, and so on. The RAM or the hard disk drive stores a program. The microprocessor operates in accordance with the program, and accordingly, the server 100 or the operation terminal 200 achieve its functions. Here, the program is configured as a combination of instruction codes indicating instructions given to a computer to achieve a predetermined function.

Furthermore, some or all of the components of the server 100 or the operation terminal 200 described above may be configured from a single system large scale integration (LSI). The system LSI is a super-multifunctional LSI manufactured by integrating constituents on a single chip, and is specifically a computer system including a microprocessor, a ROM, a RAM, and so on. The RAM stores a computer program. The microprocessor operates in accordance with the computer program, and accordingly the system LSI achieves its functions.

Furthermore, some or all of the components of the server 100 or the operation terminal 200 described above may be configured as an IC card detachable from a computer or as a standalone module. The IC card or the module is a computer system including a microprocessor, a ROM, a RAM, and so on. The IC card or the module may include the super-multifunction LSI described above. The microprocessor operates in accordance with the computer program, and accordingly the IC card or the module achieves its functions. The IC card or the module may be tamper-resistant.

Further, the present disclosure may provide a control method or an information providing method executed by the server 100 or the operation terminal 200 described above. These methods may be implemented by a computer executing a program, or may be implemented by a digital signal including the program.

Further, the present disclosure may be implemented by recording a program or a digital signal on a non-transitory computer-readable recording medium. Examples of the recording medium include a flexible disk, a hard disk, a CD-ROM, an MO, a DVD, a DVD-ROM, a DVD-RAM, a BD (Blu-ray (registered trademark) Disc), and a semiconductor memory. The program may be configured as the above-described digital signal recorded on a non-transitory recording medium.

Further, the present disclosure may be implemented by transmitting the program or the digital signal described above via a telecommunication line, a wireless or wired communication line, a network represented by the Internet, data broadcasting, or the like.

Further, the present disclosure may be directed to a computer system including a microprocessor and a memory, the memory may store a program, and the microprocessor may operate in accordance with the program.

The program or the digital signal may be implemented by another independent computer system by being recorded on the non-transitory recording medium described above and transferred, or by being transferred via the network described above or the like.

### (Others)

A modification of an embodiment of the present disclosure may be as follows.

A method executed by a computer, including:
(a) determining information including a first temperature, based on information including a first calorific value specified by a user and information indicating one or more ingredients, such that a cooking device starts cooking the one or more ingredients at a first time under a first condition to generate a dish having the first calorific value, the first condition including a condition in which the cooking device cooks the one or more ingredients at the first temperature;
(b) determining, based on a first image captured by a camera at a second time, a second calorific value of the one or more ingredients cooked from the first time to the second time under the first condition, the first image being an image of the one or more ingredients cooked from the first time to the second time under the first condition;
(c) determining, based on a second image captured by the camera at a third time, a third calorific value of the one or more ingredients cooked from the first time to the third time under the first condition, the second image being an image of the one or more ingredients cooked from the first time to the third time under the first condition; and
(d) determining information including a second temperature, based on the first calorific value, the second calorific value, and the third calorific value, such that the one or more ingredients cooked from the first time to a fourth time is cooked by the cooking device from a fifth time under a second condition to generate the dish having the first calorific value,

the second condition including a condition in which the one or more ingredients cooked from the first time to the fourth time is cooked by the cooking device at a second temperature, the fifth time being later than the fourth time or equal to the fourth time,
the fourth time being later than the second time and the third time,
the second time and the third time being later than the first time.

The above (a) is supported by, for example, the first learning model and its related description.

The above (b) and (c) are supported by, for example, the second learning model and its related description.

The above (d) is supported by, for example, the third learning model and its related description.

### Industrial Applicability

The present disclosure has an effect of enabling appropriate control of the nutrition of a dish, and is useful for a system, an apparatus, or the like that performs control related to cooking or provides information related to cooking.

### Reference Signs List

100 server
103 server control unit
104 server storage unit
105 server communication unit
110 first storage unit
111 dish list information
112 recipe information
113 basic cooking parameter information
114 basic nutritional information
120 second storage unit
121 cooking state information
121a image data
121b chemical analysis data
121c weight data
130 third storage unit
131 nutritional information
131a target nutritional information
131b first nutritional information
131c second nutritional information
131d third nutritional information
140 fourth storage unit
141 initial cooking parameter information
142 modified cooking parameter information
150 model storage unit
150a learning model set
151 first learning model
152 second learning model
153 third learning model
154 fourth learning model
200 operation terminal
201 input unit
202 display unit
203 terminal control unit
204 terminal storage unit
205 terminal communication unit
300 cooking device
303 cooking control unit
304 cooking storage unit
305 cooking communication unit
310 cooking state acquisition unit
311 weight measurement unit
312 image capturing unit
313 chemical analysis unit
320 cooking unit
321 pressure adjustment unit
322 temperature adjustment unit
323 time adjustment unit
1000 information processing system
1031 input acquisition unit
1032 parameter acquisition unit
1033 nutritional information acquisition unit
1034 parameter modification unit
1035 processing unit
1037 parameter output unit

## Claims

1. A control method executed by a computer to control a cooking device that cooks an item to be cooked, the control method comprising:
acquiring target nutritional information indicating a target for one or more numerical values related to nutrition of a dish obtained by cooking the item to be cooked;
acquiring cooking parameter information including one or more cooking parameters used for cooking by the cooking device, based on the acquired target nutritional information;
acquiring cooking-in-progress nutritional information indicating the one or more numerical values related to the nutrition of the item to be cooked when the cooking by the cooking device is being performed;
changing the cooking parameter information to modified cooking parameter information including one or more modified cooking parameters, based on the target nutritional information and the cooking-in-progress nutritional information; and
outputting a control signal including the modified cooking parameter information.

2. The control method according to claim 1, wherein
the acquiring of the cooking-in-progress nutritional information
acquires, as first nutritional information, the cooking-in-progress nutritional information at a time point when a first time period elapses after a start of the cooking by the cooking device, and
acquires, as second nutritional information, the cooking-in-progress nutritional information at a time point when a second time period elapses after the first time period has elapsed since the start of the cooking by the cooking device, and
the changing of the cooking parameter information
changes the cooking parameter information to the modified cooking parameter information, based on the target nutritional information and a difference between the first nutritional information and the second nutritional information.

3. The control method according to claim 1, further comprising:
acquiring third nutritional information indicating the one or more numerical values related to the nutrition of the dish obtained by cooking according to the modified cooking parameter information by the cooking device.

4. The control method according to claim 1, wherein
the changing of the cooking parameter information
acquires candidates for the modified cooking parameter information, based on the target nutritional information and the cooking-in-progress nutritional information, and
selects, from among the candidates, a candidate whose difference from the cooking parameter information is less than or equal to a threshold or a candidate closest to the cooking parameter information, as the modified cooking parameter information.

5. The control method according to claim 3, wherein
the acquiring of the third nutritional information
acquires the third nutritional information at least by inputting the modified cooking parameter information to a learning model, and
the learning model is trained by machine learning such that, at least in response to an input of one or more cooking parameters changed during the cooking by the cooking device, the one or more numerical values related to the nutrition of the dish obtained by cooking according to the one or more cooking parameters by the cooking device are output.

6. The control method according to claim 1, wherein
the acquiring of the cooking-in-progress nutritional information
acquires the cooking-in-progress nutritional information by inputting, to a learning model, at least one of an image of the item to be cooked, a weight of the item to be cooked, and an amount of a chemical component contained in the item to be cooked when the cooking by the cooking device is being performed.

7. The control method according to claim 6, wherein
the learning model is
trained by machine learning such that, in response to an input of at least one of an image of one or more food ingredients being cooked by the cooking device, a weight of the one or more food ingredients, and an amount of a chemical component contained in the one or more food ingredients, the one or more numerical values related to the nutrition of the one or more food ingredients are output.

8. The control method according to any one of claims 1 to 7, wherein
the one or more cooking parameters include a parameter indicating a temperature used for the cooking by the cooking device, and a parameter indicating a time used for the cooking by the cooking device.

9. The control method according to claim 8, wherein
the one or more cooking parameters further include a parameter indicating a pressure used for the cooking by the cooking device.

10. An information providing method for a computer to provide information related to cooking of an item to be cooked by a cooking device, the information providing method comprising:
receiving, in response to an operation by a user, target nutritional information indicating a target for one or more numerical values related to nutrition of a dish obtained by cooking the item to be cooked; and
outputting final nutritional information indicating the one or more numerical values related to the nutrition of the dish, the final nutritional information being derived based on the target nutritional information and cooking-in-progress nutritional information, the cooking-in-progress nutritional information indicating the one or more numerical values related to the nutrition of the item to be cooked when the item to be cooked is being cooked by the cooking device.

11. A control system for controlling a cooking device that cooks an item to be cooked, the control system comprising:
an input acquirer that acquires target nutritional information indicating a target for one or more numerical values related to nutrition of a dish obtained by cooking the item to be cooked;
a parameter acquirer that acquires cooking parameter information including one or more cooking parameters used for cooking by the cooking device, based on the acquired target nutritional information;
a nutritional information acquirer that acquires cooking-in-progress nutritional information indicating the one or more numerical values related to the nutrition of the item to be cooked when the cooking by the cooking device is being performed;
a parameter modifier that modifies the cooking parameter information to modified cooking parameter information including one or more modified cooking parameters, based on the target nutritional information and the cooking-in-progress nutritional information; and
a parameter outputter that outputs a control signal including the modified cooking parameter information to the cooking device.

12. An information providing system for providing information related to cooking of an item to be cooked by a cooking device, the information providing system comprising:
an inputter that receives, in response to an operation by a user, target nutritional information indicating a target for one or more numerical values related to nutrition of a dish obtained by cooking the item to be cooked; and
an outputter that outputs final nutritional information indicating the one or more numerical values related to the nutrition of the dish, the final nutritional information being derived based on the target nutritional information and cooking-in-progress nutritional information, the cooking-in-progress nutritional information indicating the one or more numerical values related to the nutrition of the item to be cooked when the item to be cooked is being cooked by the cooking device.

13. A program for controlling a cooking device that cooks an item to be cooked, the program causing a computer to execute:
acquiring target nutritional information indicating a target for one or more numerical values related to nutrition of a dish obtained by cooking the item to be cooked;
acquiring cooking parameter information including one or more cooking parameters used for cooking by the cooking device, based on the acquired target nutritional information;
acquiring cooking-in-progress nutritional information indicating the one or more numerical values related to the nutrition of the item to be cooked when the cooking by the cooking device is being performed;
changing the cooking parameter information to modified cooking parameter information including one or more modified cooking parameters, based on the target nutritional information and the cooking-in-progress nutritional information; and
outputting a control signal including the modified cooking parameter information.

14. A program for providing information related to cooking of an item to be cooked by a cooking device, the program causing a computer to execute:
receiving, in response to an operation by a user, target nutritional information indicating a target for one or more numerical values related to nutrition of a dish obtained by cooking the item to be cooked; and
outputting final nutritional information indicating the one or more numerical values related to the nutrition of the dish, the final nutritional information being derived based on the target nutritional information and cooking-in-progress nutritional information, the cooking-in-progress nutritional information indicating the one or more numerical values related to the nutrition of the item to be cooked when the item to be cooked is being cooked by the cooking device.
